(19) Europäisches Patentamt
European Patent Office
Office européen des brevets

(11) EP 2 795 691 B1

(12) **EUROPÄISCHE PATENTSCHRIFT**

(45) Veröffentlichungstag und Bekanntmachung des Hinweises auf die Patenterteilung:
**20.02.2019 Patentblatt 2019/08**

(21) Anmeldenummer: **12812903.8**

(22) Anmeldetag: **11.12.2012**

(51) Int Cl.:
***H01L 51/54*** *(2006.01)*

(86) Internationale Anmeldenummer:
**PCT/EP2012/075112**

(87) Internationale Veröffentlichungsnummer:
**WO 2013/092313 (27.06.2013 Gazette 2013/26)**

(54) **ORGANISCHE MOLEKÜLE FÜR OLEDS UND ANDERE OPTO-ELEKTRONISCHE VORRICHTUNGEN**

ORGANIC MOLECULES FOR OLEDS AND OTHER OPTOELECTRONIC DEVICES

MOLÉCULES ORGANIQUES POUR OLED (DIODES ÉLECTROLUMINESCENTES ORGANIQUES) ET AUTRES DISPOSITIFS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 DE 102011089687**

(43) Veröffentlichungstag der Anmeldung:
**29.10.2014 Patentblatt 2014/44**

(73) Patentinhaber: **cynora GmbH**
**76646 Bruchsal (DE)**

(72) Erfinder:
- **HUPFER, Alexander**
  **93326 Abendsberg (DE)**
- **YERSIN, Hartmut**
  **93161 Sinzing (DE)**

(74) Vertreter: **Hoppe, Georg Johannes**
**Darani Anwaltskanzlei**
**Beuckestrasse 20**
**14163 Berlin (DE)**

(56) Entgegenhaltungen:
**WO-A1-2011/147522 DE-A1-102008 033 563**

- **BALUSCHEV S ET AL: "Two pathways for photon upconversion in model organic compound systems", JOURNAL OF APPLIED PHYSICS, AMERICAN INSTITUTE OF PHYSICS. NEW YORK, US, Bd. 101, Nr. 2, 16. Januar 2007 (2007-01-16), Seiten 23101-023101, XP012097348, ISSN: 0021-8979, DOI: 10.1063/1.2409618**
- **ENDO AYATAKA ET AL: "Efficient up-conversion of triplet excitons into a singlet state and its application for organic light emitting diodes", APPLIED PHYSICS LETTERS, AIP, AMERICAN INSTITUTE OF PHYSICS, MELVILLE, NY, US, Bd. 98, Nr. 8, 24. Februar 2011 (2011-02-24), Seiten 83302-83302, XP012140060, ISSN: 0003-6951, DOI: 10.1063/1.3558906**
- **YI-TING LEE ET AL: "Solid-state highly fluorescent diphenylaminospirobifluorenylfumaronitrile red emitters for non-doped organic light-emitting diodes", CHEMICAL COMMUNICATIONS, Nr. 2, 1. Januar 2008 (2008-01-01), Seite 217, XP055053214, ISSN: 1359-7345, DOI: 10.1039/b711157f**
- **IGNACY GRYCZYNSKI ET AL: "Thermal deactivation of the lowest singlet and triplet excited states of acridine dyes in poly(vinyl alcohol) films", JOURNAL OF PHOTOCHEMISTRY, Bd. 31, Nr. 2-3, 1. Dezember 1985 (1985-12-01), Seiten 265-272, XP055053212,**
- **XING XING ET AL: "An alternative way to use the triplet energy of fluorescent dyes in organic light-emitting devices via an external iodide", ORGANIC ELECTRONICS, ELSEVIER, AMSTERDAM, NL, Bd. 13, Nr. 1, 23. Oktober 2011 (2011-10-23), Seiten 195-198, XP028341764, ISSN: 1566-1199, DOI: 10.1016/J.ORGEL.2011.10.019 [gefunden am 2011-11-16]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 795 691 B1

- WEN-YI HUNG ET AL: "An ambipolar host material provides highly efficient saturated red PhOLEDs possessing simple device structures", PHYSICAL CHEMISTRY CHEMICAL PHYSICS, vol. 10, no. 38, 1 January 2008 (2008-01-01), page 5822, XP055142437, ISSN: 1463-9076, DOI: 10.1039/b811356b

## Beschreibung

Die vorliegende Erfindung betrifft die Verwendung von speziellen organischen Farbstoffen ohne Metallzentren als Emitter in OLEDs (organic light emitting diodes) und in anderen opto-elektronischen Anordnungen.

## Einleitung

Zur Zeit lässt sich erkennen, dass OLED-Anordnungen bereits jetzt wirtschaftlich bedeutend sind, da eine Massenfertigung in Kürze zu erwarten ist. Derartige OLEDs bestehen vorwiegend aus organischen Schichten, die auch flexibel und kostengünstig zu fertigen sind. OLED-Bauelemente lassen sich großflächig als Beleuchtungskörper, aber auch klein als Pixel für Displays gestalten.

Gegenüber herkömmlichen Technologien, wie etwa Flüssigkristall-Displays (LCDs), Plasma-Displays oder Kathodenstrahlenröhren (CRTs), weisen OLEDs zahlreiche Vorteile auf, wie eine geringe Betriebsspannung von einigen Volt, eine dünne Struktur von nur einigen hundert nm, hoch-effizient selbst-leuchtende Pixel, einen hohen Kontrast und eine gute Auflösung sowie die Möglichkeit, alle Farben darzustellen. Weiterhin wird in einem OLED Licht beim Anliegen elektrischer Spannung direkt erzeugt, anstelle es nur zu modulieren.

Einen Überblick über die Funktion von OLEDs findet sich beispielsweise bei H. Yersin, Top. Curr. Chem. 2004, 241, 1 und H. Yersin, "Highly Efficient OLEDs with Phosphorescent Materials"; Wiley-VCH, Weinheim, Germany, 2008.

Seit den ersten Berichten über OLEDs (siehe z.B. Tang et al., Appl. Phys. Lett. 1987, 51, 913) sind diese Vorrichtungen besonders im Hinblick auf die eingesetzten Emittermaterialien weiterentwickelt worden, wobei insbesondere in den letzten Jahren sogenannte Triplett- oder auch andere phosphoreszierende Emitter von Interesse sind.

OLEDs werden in der Regel in Schichtenstrukturen realisiert. Zum besseren Verständnis ist in Fig. 1 ein prinzipieller Aufbau eines OLEDs gezeigt. Aufgrund der angelegten äußeren Spannung an einer transparenten Indium-Zinn-Oxid-Anode (ITO) und einer dünnen Metall-Kathode werden von der Anode positive Löcher und von der Kathode negative Elektronen injiziert. Diese verschieden geladenen Ladungsträger gelangen über Zwischenschichten, die auch aus hier nicht gezeichneten Loch- bzw. Elektronen-Blockierschichten bestehen können, in die Emissionsschicht. Dort treffen die entgegengesetzt geladenen Ladungsträger an oder in der Nähe von dotierten Emitter-Molekülen zusammen und rekombinieren. Die Emitter-Moleküle sind in der Regel in Matrizen bestehend aus kleinen Molekülen oder Polymermatrizen (in z. B. 2 bis 10 Gew.-%) eingelagert, wobei die Matrix-Materialien so gewählt sind, dass sie auch einen Loch- und Elektronentransport ermöglichen. Durch die Rekombination entstehen Exzitonen (= Anregungszustände), die ihre Überschussenergie auf die jeweilige elektro-lumineszierende Verbindung übertragen. Diese Verbindung kann daraufhin in einen bestimmten elektronischen Anregungszustand übergehen, der dann möglichst vollständig und unter weitgehender Vermeidung strahlungsloser Desaktivierungsprozesse durch Lichtemission wieder in den zugehörigen Grundzustand übergeht.

Als elektronischer Anregungszustand, der auch durch Energieübertragung von einem geeigneten Vorläufer-Exziton gebildet werden kann, kommt, von wenigen Ausnahmen abgesehen, entweder ein Singulett- oder Triplett-Zustand in Betracht. Da beide Zustände aufgrund der Spinstatistik in der Regel im Verhältnis 1:3 besetzt werden, ergibt sich, dass bei einer Emission aus dem Singulett-Zustand, die als Fluoreszenz bezeichnet wird, nach dem Stand der bisherigen Technik, nur maximal 25 % der erzeugten Exzitonen zur Emission führen. Dagegen können bei einer Triplett-Emission, die als Phosphoreszenz bezeichnet wird, sämtliche Exzitonen ausgenutzt, umgewandelt und als Licht emittiert werden (Triplett-Harvesting), so dass in diesem Fall die Innere Quantenausbeute den Wert von 100 % erreichen kann, sofern der mitangeregte und energetisch über dem Triplett-Zustand liegende Singulett-Zustand vollständig in den Triplett-Zustand relaxiert (Inter-System-Crossing, ISC) und strahlungslose Konkurrenzprozesse bedeutungslos bleiben. Somit sind Triplett-Emitter nach dem bisherigen Stand der Technik effizientere Elektro-Luminophore und besser geeignet als rein organische Singulett-Emitter, in einer organischen Leuchtdiode für eine hohe Lichtausbeute zu sorgen.

Bei den für das Triplett-Harvesting geeigneten Triplett-Emittern werden in der Regel Übergangsmetall-Komplexverbindungen eingesetzt, in denen das Metall aus der dritten Periode der Übergangsmetalle gewählt wird. Hierbei handelt es sich vorwiegend um sehr teure Edelmetalle wie Iridium, Platin oder auch Gold. (Siehe dazu auch H. Yersin, Top. Curr. Chem. 2004, 241, 1 und M. A. Baldo, D. F. O’Brien, M. E. Thompson, S. R. Forrest, Phys. Rev. B 1999, 60, 14422)

Die bisher bekannten phosphoreszierenden, metall-organischen Triplett-Emitter in OLEDs weisen allerdings einen Nachteil auf, der darin besteht, dass diese Komplexe in elektronisch angeregten Zuständen häufig chemisch reaktiver als in den Grundzuständen sind. Verantwortlich dafür sind in der Regel Metall-Ligand-Brüche. Daher ist die Langzeitstabilität dieser Emitter-Materialien in vielen Fällen unzureichend. (T. Sajoto, P. I. Djurovich, A. B. Tamayo, J. Oxgaard, W. A. Goddard III, M. E. Thompson; J. Am. Chem. Soc. 2009, 131, 9813). Infolgedessen wird angestrebt, Emitter-Moleküle ohne Metallzentren und mit hoher Emissionsquantenausbeute zu entwickeln, wobei die Emitter-Moleküle darüber hinaus auch sämtliche Singulett- und Triplett-Exzitonen in Licht umwandeln sollen. OLEDs unter Verwendung derartiger Emitter sollten einen hohen Wirkungsgrad zeigen und darüber hinaus eine längere Lebensdauer der

optoelektronischen Vorrichtung ermöglichen. Zusammenfassend lässt sich der Stand der Technik so beschreiben, dass die bisher bekannten an sich effizienten Triplett-Emitter die Nachteile aufweisen, dass

- teure Edelmetall-Moleküle verwendet werden müssen und dass
- diese auf der Basis von metall-organischen Komplexen aufgebauten Emitter in vielen Fällen nur unzureichende Langzeitstabilität aufweisen.

Aus dem Artikel Wen-Yi Hung et al., Physical Chemistry Chemical Physics, Band 10, Nr. 38, 1. Januar 2008, Seite 5822-5825 ist ein elektrophosphoreszente Vorrichtung bekannt, die ein Donor-Akzeptor-Hostmaterial und einen Iridiumkomplex aufweist.

In X. Xing et al., Organic Electronics 13 (2012), 195-198 werden OLEDs beschrieben, welche eine organische Jod-Verbindung in der Emitterschicht aufweisen.

**Beschreibung der Erfindung**

Überraschender Weise lässt sich die oben beschriebene Problematik durch die vorliegende Erfindung wesentlich verbessern bzw. lösen, wobei organische Moleküle (Farbstoffe, Emitter-Moleküle) zur Verwendung kommen, die bestimmte elektronische Strukturen bzw. Singulett-Triplett-Energieabstände aufweisen und die erfindungsgemäß durch Veränderungen in der unmittelbaren Umgebung der Emitter modifiziert werden. Dieses Verfahren des hier erstmals vorgeschlagenen "Singulett-Harvestings für organische Emitter" soll im Folgenden unter Verwendung der Fig. 2 kurz beschrieben werden:

Fig. 2a zeigt ein (vereinfachtes) Energieniveauschema für ein typisches, rein organisches Molekül, das einen $\Delta E(S_1-T_1)$-Wert zwischen dem untersten angeregten Singulett-($S_1$) und dem darunter liegenden Triplett-Zustand ($T_1$) von deutlich größer als 3000 $cm^{-1}$ aufweist.

Anhand dieses Schemas lassen sich die photo-physikalischen Elektrolumineszenz-Eigenschaften dieser Moleküle erläutern. Die Loch-Elektron-Rekombination, wie sie beispielsweise in einem optoelektronischen Bauelement erfolgt, führt im statistischen Mittel zu 25 % zur Besetzung des Singulett-Zustandes und zu 75 % zur Besetzung der drei Unterzustände des Triplett-Zustandes. Da der Emissions-Übergang aus dem Triplett- $T_1$ in den Singulett- $S_0$ Zustand bei organischen Molekülen wegen der geringen Spin-Bahn-Kopplung stark spin-verboten ist, wird die in den Triplett gelangende Anregungsenergie in der Regel strahlungslos in Wärme umgewandelt und ist damit für die Licht-Erzeugung durch Elektro-Lumineszenz verloren. Der besetzte Singulett-Zustand kann allerdings eine effektive Emission (Fluoreszenz) zeigen, weil es sich um einen spin-erlaubten Singulett-Singulett-Übergang handelt. In diesem Zusammenhang ist es wichtig zu erwähnen, dass der strahlungslose Relaxationsprozess aus dem $S_1$-Zustand in den $T_1$-Zustand, das sogenannte Inter-System-Crossing (ISC), ebenfalls wegen der geringen Spin-Bahn-Kopplung stark verboten ist. Andernfalls würde keine Fluoreszenz beobachtbar sein. Für die Zeitkonstanten bedeutet das, dass $\tau_1$(ISC) um ein Vielfaches länger ist als die Fluoreszenzlebensdauer, die im Bereich von einer bis zu einigen Nanosekunden für $\tau(S_1)$ liegt.

Erfindungsgemäß lassen sich die oben beschriebenen Nachteile des Standes der Technik vermeiden. Das gelingt unter Kombination von zwei Schritten:

I. Es werden organische Moleküle mit hoher Emissionsquantenausbeute (größer 50 %) bereitgestellt, deren Energiedifferenz zwischen dem Singulett $S_1$ und dem Triplett $T_1$ so klein ist, so dass bei Raumtemperatur eine thermische Rückbesetzung vom Triplett $T_1$ in den Singulett $S_1$ möglich ist, wodurch die Triplett-Anregung über den Singulett $S_1$ Zustand in Licht umgewandelt werden kann. Das gelingt erfindungsgemäß unter Verwendung rein organischer Moleküle, wie etwa unter Verwendung von organischen Molekülen der Formeln I, II, III, IV und/oder V.

II. Die extrem lange Inter-System-Crossing Zeitkonstante ($\tau$(ISC)) von rein organischen Molekülen wird, um eine ausreichend schnelle thermische Rückbesetzung, das sogenannte Up-Intersystem-Crossing, zu ermöglichen, um einige Größenordnungen verkürzt. Das ist durch eine Verstärkung der Spin-Bahn-Kopplung insbesondere durch zusätzliches Einbringen von Atomen oder Molekülen möglich, die eine hohe Spin-Bahn-Kopplung tragen. Die Verstärkung der Spin-Bahnkopplung kann auch durch kovalente Bindung eines Zusatzstoffes an das Emitter-Molekül erfolgen. Dem Chemiker sind diese Effekte als "externer" bzw. "innerer" Schweratomeffekt" bekannt. Dieses Verfahren wird weiter untern erläutert.

Unter diesen beiden, gemeinsam einzusetzenden Strategien lassen sich - wie anhand der Fig. 2 b erläutert wird - die bei der Elektro-Lumineszenz-Anregung besetzten Triplett- und Singulett-Anregungen sammeln und über den Singulett-Zustand $S_1$ in Licht umwandeln. Dieses hier erstmals beschriebene Verfahren des Singulett-Harvesting-Effektes für organische Moleküle wird im Folgenden genauer erläutert.

Demgemäß sieht die Erfindung gemäß Anspruch 1 in einem Aspekt eine Zusammensetzung, insbesondere zur Benutzung in einer optoelektronischen Vorrichtung vor, die

- ein organisches Emitter-Molekül mit einem untersten angeregten Singulett- ($S_1$) und einem darunter liegenden Triplett-Zustand ($T_1$) aufweist, wobei der $\Delta E(S_1-T_1)$-Wert des organischen Moleküls kleiner als 3000 $cm^{-1}$ (bevorzugt kleiner als 2500 $cm^{-1}$) ist, insbesondere zwischen 10 $cm^{-1}$ und kleiner als 3000 $cm^{-1}$ liegt, und
- ein optisch inertes Atom oder Molekül aufweist, das derart mit dem organischen Molekül in Wechselwirkung tritt, dass die Inter-System-Crossing Zeitkonstante für die thermische Rückbesetzung, d. h. die Up-Inter-System-Crossing Zeitkonstante, des organischen Moleküls auf kleiner als 300 ms, bevorzugt auf kleiner als 1 ms, besonders bevorzugt auf kleiner als 1 $\mu$s reduziert wird. Dies erfolgt durch ein optisch inertes Atom oder Molekül oder Molekül-Komponenten, das oder die eine hohe Spin-Bahn-Kopplung aufweist/aufweisen. Diese lässt sich durch die Spin-Bahn-Kopplungskonstante beschreiben, die höher als etwa 200 $cm^{-1}$, bevorzugt höher als 1000 $cm^{-1}$ und besonders bevorzugt höher als 2000 $cm^{-1}$, ganz besonders bevorzugt größer 4000 $cm^{-1}$ sein sollte.

Die Begriffe "Spin-Bahn-Kopplungskonstante", "Inter-System-Crossing Zeitkonstante" und "Up-Inter-System-Crossing Zeitkonstante" sind Fachbegriffe, die in der photophysikalischen Literatur verwendet werden und daher dem Fachmann bekannt sind.

**Moleküle mit kleinen $\Delta E(S_1-T_1)$-Abständen**

In Fig. 2 b ist ein Energieniveau-Diagramm für ein organisches Molekül mit kleiner Energiedifferenz $\Delta E(S_1-T_1) < 3000$ $cm^{-1}$ dargestellt. Diese Energiedifferenz ist klein genug, um eine thermische Rückbesetzung des $S_1$-Zustandes aus dem $T_1$-Zustand gemäß einer Boltzmann-Verteilung bzw. gemäß der thermischen Energie $k_BT$, und damit eine thermisch aktivierte Lichtemission aus dem $S_1$-Zustand zu ermöglichen. Dieser Vorgang, der als thermisch aktivierte (verzögerte) Fluoreszenz bezeichnet wird, wird vereinfacht durch Gleichung (1) gesteuert

$$\mathrm{Int}(S_1 \rightarrow S_0) / \mathrm{Int}(T_1 \rightarrow S_0) = k(S_1) / k(T_1) \exp(-\Delta E/k_BT) \qquad (1)$$

Hierin stellt $\mathrm{Int}(S_1 \rightarrow S_0) / \mathrm{Int}(T_1 \rightarrow S_0)$ das Intensitätsverhältnis der Emissionen aus dem $S_1$-Zustand bzw. dem $T_1$-Zustand dar. $k_B$ ist die Boltzmann-Konstante und T die absolute Temperatur. $k(S_1) / k(T_1)$ ist das Ratenverhältnis der Übergangsprozesse aus dem Singulett $S_1$ und aus dem Triplett $T_1$ in den elektronischen Grundzustand $S_0$. Für organische Moleküle liegt dieses Verhältnis in der Regel zwischen $10^6$ bis $10^{10}$. Bevorzugt sind Moleküle mit einem Ratenverhältnis von etwa $10^7$, besser von etwa $10^9$, besonders bevorzugt von etwa $10^{10}$. $\Delta E$ steht für die Energiedifferenz $\Delta E_2(S_1-T_1)$ gemäß Figur 2b.

Durch den beschriebenen Prozess der thermischen Rückbesetzung wird aus dem besetzten Triplett ein Emissionskanal über den Singulett-Zustand $S_1$ geöffnet. Da der Übergang aus dem $S_1$- in den $S_0$- Zustand stark erlaubt ist, wird die sonst verlorene Triplett-Anregungsenergie praktisch vollständig als Lichtemission über den Singulett-Zustand gewonnen. Dieser Effekt ist bei vorgegebener Temperatur, z. B. bei Raumtemperatur, umso ausgeprägter, je kleiner die Energiedifferenz $\Delta E$ ist. Daher sind organische Moleküle bevorzugt, die einen $\Delta E = \Delta E(S_1-T_1)$-Wert zwischen dem untersten angeregten Singulett- und dem darunter liegenden Triplett-Zustand von kleiner als 3000 $cm^{-1}$, besser kleiner als 2500 $cm^{-1}$ oder 1500 $cm^{-1}$, bevorzugt von kleiner als 1000 $cm^{-1}$ aufweisen.

Anhand eines Zahlenbeispiels soll dieser Effekt erläutert werden. Bei einer Energiedifferenz von $\Delta E = 1300$ $cm^{-1}$ ergibt sich für Raumtemperaturanwendungen (T = 300 K) mit $k_BT = 210$ $cm^{-1}$ und einem Ratenverhältnis von $10^8$ ein Intensitätsverhältnis der Singulett- zur Triplett-Emission gemäß Gleichung (1) von ca. $2 \cdot 10^5$. Das heißt, der Singulett-Emissionsprozess dominiert für ein Molekül mit diesen Beispielwerten extrem.

Die Anwendbarkeit der Gleichung (1) erfordert erfindungsgemäß die Verwendung von Zusatzstoffen, die die Spin-Bahn-Kopplung erhöhen (für detaillierte Ausführungen siehe z. B. weiter unten). Diese Zusatzstoffe, d. h. optisch inerte Atome oder Moleküle der Zusammensetzung, treten derart mit den organischen Emitter-Molekülen in Wechselwirkung, dass die mittlere (thermalisierte) Emissionslebensdauer der beiden Zustände $S_1$ und $T_1$ des organischen Moleküls stark reduziert wird. Bevorzugt sind Zusammensetzungen derart, dass die Emissionslebensdauer auf kleiner 500 ms, bevorzugt auf kleiner 1 ms, besonders bevorzugt auf kleiner 20 $\mu$s, stärker bevorzugt auf kleiner 10 $\mu$s und ganz besonders bevorzugt auf kleiner 1 $\mu$s reduziert wird. Wesentlich ist, dass die Zeit der thermisch aktivierten Rückbesetzung aus dem $T_1$-Zustand kürzer ist (z. B. um den Faktor 5) als die Phosphoreszenzabklingzeit $\tau(T_1)$ ohne thermische Rückbesetzung. Diese Abklingzeit $\tau(T_1)$ lässt sich einfach mit käuflichen Messgeräten bei tiefen Temperaturen, z. B. bei 77 K, bestimmen.

Zusammenfassend lassen sich also unter Verwendung dieses "Singulett-Harvesting-Verfahrens für organische Moleküle" im Idealfall nahezu sämtliche, d. h. maximal 100 % der Exzitonen erfassen und über eine Singulett-Emission in Licht umwandeln. Darüber hinaus gelingt es, die Emissionsabklingzeit deutlich unter den Wert für rein organische Triplett-Emitter, der bei einigen Sekunden liegen kann, zu verkürzen. Daher ist die erfindungsgemäße Zusammensetzung

für optoelektronische Bauelemente besonders geeignet.

Organische Moleküle mit den oben beschriebenen Eigenschaften, d. h. mit kleiner Singulett-Triplett-Energiedifferenz $\Delta E$ ($S_1$-$T_1$), sind bevorzugt organische Moleküle mit folgenden generellen Formeln I bis III.

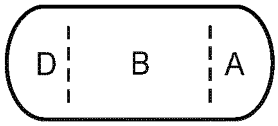


Formel I

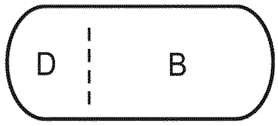


Formel II

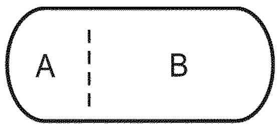


Formel III

Hierin bedeutet D eine chemische Gruppe oder steht für einen Substituenten mit elektronen-schiebender Wirkung (D, Donator-Wirkung). Substituenten dieser Art können einfach, zweifach oder mehrfach vorhanden sein. Sie können gleich oder verschieden sein.

A steht für eine chemische Gruppe oder einen Substituenten mit elektronen-ziehenden Eigenschaft (A, Akzeptor-Wirkung). Substituenten dieser Art können einfach, zweifach oder mehrfach vorhanden sein. Sie können gleich oder verschieden sein.

Das Grundgerüst B ist aufgebaut aus konjugierten organischen Gruppen, die zum Beispiel aus aromatischen, heteroaromatischen und/oder konjugierten Doppelbindungen bestehen. In einer Ausführungsform kann das Grundgerüst auch eine nicht-konjugierte Gruppe darstellen. Wesentlich ist, dass die Molekülorbitale von A und B bzw. von D und B einen gleichen Raumbereich überdecken. Das Grundgerüst B selbst kann auch elektron-ziehende Wirkung haben, dann kann (z. B. in Formel I) die Substitution beidseitig D-Charakter aufweisen. Alternativ kann das Grundgerüst B selbst elektron-schiebende Wirkung haben, dann kann (z. B. in Formel I) die Substitution beidseitig A-Charakter aufweisen.

Die Formeln I bis III repräsentieren auch, dass die elektronischen Wellenfunktionen der Moleküle in den Bereichen D/B bzw. B/A überlappen. Diese Eigenschaft kann, wie weiter unten beschrieben wird, rechnerisch ermittelt werden. Der Begriff einer elektronischen Wellenfunktion ist dem Fachmann bekannt.

Beispiele für Donatoren D:

-O(-), -NH-Alkylgruppe, -N-(Alkylgruppe)$_2$ -$NH_2$, -OH, -O-Alkylgruppe, -NH(CO)Alkylgruppe, -O(CO), - Alkylgruppe, -Arylgruppe, -heterocyklische Gruppen -(CH)=C-(Alkylgruppe)$_2$, -Phenoxazinyl, -Phenothiazinyl, -Carbazolyl, -Dihydrophenazinyl, -N(R')(R") mit (R', R" = H, Alkyl, Aryl, halogeniertes Alkyl, halogeniertes Aryl), alle Aryl- und heterocyklischen Gruppen können mit Alkyl- und/oder oder Arylgruppen substituiert sein, alle Alkylgruppen können auch mit F, Cl, Br und/oder I substituiert sein.

Beispiele für Akzeptoren A:

-Halogen, -(CO)H, -(CO)-Alkylgruppe, -(CO)O-Alkylgruppe, -(CO)OH, -(CO)Cl, -$CF_3$, -$BF_2$, -CN, -S(O)$_2$OH,

-S(O)$_2$O-Alkyl, -$NH_3^+$, -N(Alkylgruppe)$_3^+$, -$NO_2$, halogeniertes Alkyl, -B(R')(R") mit (R', R" = H, Alkyl, Aryl, halogeniertes Alkyl, halogeniertes Aryl).

Aufbau des Grundgerüstes B:

B ist aufgebaut aus konjugierten organischen Gruppen, die aus aromatischen, heteroaromatischen und/oder konjugierten Doppelbindungen bestehen. Bevorzugt sind Molekül-Grundgerüste B mit aromatischen oder heteroaromatischen Ringen kleiner 15, besonders bevorzugt kleiner 10, ganz besonders bevorzugt kleiner sieben. Die aromatischen oder heteroaromatischen Ringe sind chemisch direkt verknüpft oder über Alkenyl-Gruppen mit konjugierten Doppelbindungen kleiner 10, besonders bevorzugt kleiner sechs und ganz besonders bevorzugt kleiner 3 chemisch verbunden. In einer Ausführungsform kann das Grundgerüst auch eine nicht-konjugierte Gruppe darstellen. Wesentlich ist, dass die Molekülorbitale von A und B bzw. von D und B einen gleichen Raumbereich überdecken.

Die durch Formeln I bis III beschriebenen organischen Moleküle weisen $\Delta E(S_1-T_1)$-Werte zwischen dem untersten angeregten Singulett- und dem darunter liegenden Triplett-Zustand von kleiner als 3000 $cm^{-1}$, bevorzugt kleiner als 2500 $cm^{-1}$ oder 1500 $cm^{-1}$ und besonders bevorzugt kleiner 1000 $cm^{-1}$ auf. Auf Verfahren zur Messung bzw. rechnerischen Bestimmung der $\Delta E(S_1-T_1)$-Werte wird weiter unten eingegangen.

Bevorzugt sind organische Moleküle, die ohne Verwendung von Zusatzstoffen eine hohe Fluoreszenz-Quantenausbeute aus dem $S_1$-Zustand von größer 30 %, bevorzugt größer 50 %, besonders bevorzugt größer 80 % aufweisen (Bestimmung mit handelsüblichen Emissionsquantenausbeute-Messgeräten) und bei denen die Absorptionsstärken, d. h. die molaren dekadischen Extinktionskoeffizienten der Übergänge zwischen dem Grundzustand So und dem angeregten Zustand $S_1$ größer als $10^3$ l/(mol cm), bevorzugt größer $10^4$ l/(mol cm), besonders bevorzugt größer als $5x10^4$ l/(mol cm) sind (Bestimmung mit handelsüblichen Absorptionsspektrometern).

Die Beschreibung betrifft in einem weiteren Aspekt ein Verfahren zur Auswahl von organischen Molekülen der hier beschriebenen Art, deren $\Delta E(S_1-T_1)$-Wert zwischen dem untersten angeregten Singulett- ($S_1$) und dem darunter liegenden Triplett-Zustand ($T_1$) kleiner als 3000 $cm^{-1}$, bevorzugt kleiner als 2500 $cm^{-1}$ oder 1500 $cm^{-1}$, besonders bevorzugt kleiner als 1000 $cm^{-1}$ ist.

Die Bestimmung des $\Delta E(S_1-T_1)$-Wertes kann sowohl durch quantenmechanische Berechnungen mittels bekannter Computerprogramme durchgeführt werden (z. B. unter Ausführung von TDDFT-Rechnungen, z. B. mit käuflich erwerbbaren Gaussian 09 oder ADF-Amsterdam Density Functional Software Programmen) oder - wie weiter unten erläutert wird - experimentell bestimmt werden. Orientierende Informationen lassen sich bereits aus vergleichsweise einfach durchzuführenden DFT-Rechnungen gewinnen (z. B. mit käuflich erwerbbaren Gaussian 09 oder ADF-Amsterdam Density Functional Software Programm). Hier zeigen z. B. die Grenzorbitale HOMO-2, HOMO-1, HOMO, LUMO, LUMO+1 und LUMO+2 Tendenzen der zu erwartenden Charge-Transfer-Eigenschaften bezüglich der untersten angeregten Energiezustände der Moleküle. Auf diese Eigenschaften wird im nächsten Abschnitt eingegangen werden.

Die Energiedifferenz $\Delta E(S_1-T_1)$, insbesondere der durch Formeln I bis III beschriebenen organischen Moleküle, lässt sich näherungsweise quantenmechanisch durch das mit dem Faktor 2 multiplizierte sogenannte Austauschintegral beschreiben. Dessen Wert hängt direkt ab von der Überlappung der Molekülorbitale im D-seitigen Bereich von B mit den Molekülorbitalen im A-seitigen Bereich von B (Formel I). Entsprechend wird der Wert des Austauschintegrals durch die Überlappung der Wellenfunktionen im D-B-Bereich (Formel II) bzw. im A-D-Bereich (Formel III) mit denen im B-Bereich bestimmt. Diese Molekülorbitale sind wegen der oben beschriebenen Eigenschaften von D bzw. A über unterschiedliche Raumbereiche verteilt (teil-delokalisiert über $\pi$- bzw. $\pi^*$-Molekülorbitale). Das heißt, ein elektronischer Übergang zwischen den verschiedenen Molekülorbitalen repräsentiert einen sogenannten Charge-Transfer (CT)-Übergang oder einen Übergang mit CT-Beteiligung innerhalb des Moleküls. Mit einem organischen Molekül, das einen entsprechenden CT-Charakter bezüglich der untersten angeregten Molekülorbitale aufweist, lässt sich das angestrebte kleine Austauschintegral und damit die kleine Energiedifferenz $\Delta E(S_1-T_1)$ erzielen, wobei auch die molaren dekadischen Extinktionskoeffizienten für den elektronischen Übergang vom $S_0$ zum $S_1$ in dem oben definierten bevorzugten Bereich liegen. Mit anderen Worten, $\Delta E(S_1-T_1)$ kann über die Stärken der elektronen-schiebenden bzw. -ziehenden Substituenten/Gruppen des organischen Moleküls verändert werden. Aufgrund dieser photophysikalischen (quantenmechanischen) Eigenschaften lassen sich die erfindungsgemäßen Energiedifferenzen mit $\Delta E(S_1-T_1)$ kleiner 3000 $cm^{-1}$ oder kleiner 2500 $cm^{-1}$ oder kleiner 1500 $cm^{-1}$ oder kleiner 1000 $cm^{-1}$ erreichen.

Die Bestimmung des $\Delta E(S_1-T_1)$-Wertes kann experimentell folgendermaßen erfolgen:

Für ein vorgegebenes organisches Molekül lässt sich der Energieabstand $\Delta E(S_1-T_1) = \Delta E$ unter Verwendung der oben angegebenen Gleichung (1) einfach bestimmen. Eine Umformung ergibt:

$$\ln\{Int(S_1 \rightarrow S_0)/Int(T_1 \rightarrow S_0)\} = \ln\{k(S_1)/k(T_1)\} - (\Delta E/k_B)(1/T) \quad (2)$$

Für die Messung der Intensitäten $Int(S_1 \rightarrow S_0)$ und $Int(T_1 \rightarrow S_0)$ kann jedes handelsübliche Spektralphotometer verwendet werden. Eine graphische Auftragung der bei verschiedenen Temperaturen gemessenen (logarithmierten) Intensitätsverhältnisse $\ln\{Int(S_1 \rightarrow S_0)/Int(T_1 \rightarrow S_0)\}$ gegen den Kehrwert der absoluten Temperatur T ergibt in der Regel eine Gerade. Die Messung wird in einem Temperaturbereich von Raumtemperatur (300 K) bis 77 K oder bis 4,2 K durchgeführt, wobei die Temperatur mittels eines Kryostaten eingestellt wird. Die Intensitäten werden aus den (korrigierten) Spektren bestimmt, wobei $Int(S_1 \rightarrow S_0)$ bzw. $Int(T_1 \rightarrow S_0)$ die integrierten Fluoreszenz- bzw. Phosphoreszenz-Bandenintensitäten repräsentieren, welche sich mittels der zum Spektralphotometer gehörenden Programme bestimmen lassen. Die jeweiligen Übergänge (Bandenintensitäten) lassen sich leicht identifizieren, da die Triplett-Bande bei niedrigerer Energie liegt als die Singulett-Bande und mit sinkender Temperatur an Intensität gewinnt. Dabei werden die Messungen in sauerstofffreien verdünnten Lösungen (ca. $10^{-2}$ mol $l^{-1}$) oder an dünnen Filmen aus den entsprechenden Molekülen oder an mit den entsprechenden Molekülen dotierten Filmen durchgeführt. Verwendet man als Probe eine Lösung, so empfiehlt es sich, ein Lösemittel bzw. Lösemittelgemisch zu verwenden, das bei tiefen Temperaturen Gläser bildet, wie 2-Methyl-THF, THF (Tetrahydrofuran) oder aliphatische Kohlenwasserstoffe. Verwendet man als Probe einen Film, so eignet sich die Verwendung einer Matrix mit einer deutlich größeren Singulett- sowie Triplett-Energie als die der organischen Emittermoleküle, z. B. PMMA (Polymethylmethacrylat). Dieser Film kann aus Lösung aufgebracht werden. Besonders wichtig ist, dass - wie unten beschrieben - die zu analysierenden Moleküle mit den jeweiligen Zusatzstoffen verwendet werden.

Die Geradensteigung beträgt $-\Delta E/k_B$. Mit $k_B = 1{,}380\ 10^{-23}\ JK^{-1} = 0{,}695\ cm^{-1}\ K^{-1}$ lässt sich der Energieabstand direkt bestimmen.

Eine äquivalente Betrachtungsweise zeigt, dass mittels der Messung der Temperaturabhängigkeit der Emissionsabklingzeit der $\Delta E(S_1\text{-}T_1)$-Wert auch bestimmt werden kann.

Eine einfache, näherungsweise Abschätzung des $\Delta E(S_1\text{-}T_1)$-Wertes kann auch dadurch vorgenommen werden, dass bei tiefer Temperatur (z. B. 77 K oder 4.2 K unter Verwendung eines Kryostaten) die Fluoreszenz- und Phosphoreszenz-Spektren registriert werden. Der $\Delta E(S_1\text{-}T_1)$-Wert entspricht dann in Näherung der Energiedifferenz zwischen den hoch-energetischen Anstiegsflanken der Fluoreszenz- bzw. Phosphoreszenz-Bande.

Je ausgeprägter der CT-Charakter eines organischen Moleküls ist, desto stärker verändern sich die elektronischen Übergangsenergien als Funktion der Lösungsmittelpolarität. (Vergleiche z. B. J. B. Birks, Photophysics of Aromatic Molecules, Wiley-Interscience, London 1970; E. Lippert, Z Naturforsch. 10a (1955) 541) So ergibt bereits der durch eine einfache Messung zu führende Nachweis einer ausgeprägten Polaritätsabhängigkeit der Emissionsenergien einen Hinweis auf das Vorliegen eines CT-Überganges und damit einen Hinweis auf kleine $\Delta E(S_1\text{-}T_1)$-Werte.

Ferner ist es wichtig, die strahlungslosen Prozesse (Raten) zu vermindern, weil dadurch die Emissionsquantenausbeuten der Emittermoleküle erhöht werden können.

- So sind in einer Ausführungsform der Erfindung die Protonen der Emittermoleküle teilweise oder vollständig durch Deuterium ersetzt.
- Auch eine Deuterierung oder Teil-Deuterierung der Matrix der opto-elektronischen Vorrichtung kann in besonderen Fällen zu einer deutlichen Erhöhung der Emissionsquantenausbeute führen und stellt eine optionale Ausführungsform dar.
- Ein weiteres Verfahren zur Reduktion der strahlungslosen Prozesse, d.h. zur Erhöhung der Emissionsquantenausbeuten der Emittermoleküle, besteht darin, die unmittelbare Umgebung durch Wahl z. B. einer Polymer-Matrix oder einer polymer-vernetzten Matrix oder einer semi-kristallinen Matrix möglichst rigide auszugestalten. Strategien zur Polymer-Vernetzung sind dem Fachmann bekannt (siehe z. B. C. A. Zuniga, S. Barlow, S. R. Marder; Chem. Materials 2011, 23, 658-681). Die in den Beispielen verwendete Matrix, Glukose-Trehalose, erfüllt diese Anforderung der Rigidität.

**Zusatzstoffe / Verringerung der Inter-System-Crossing-Zeitkonstante**

Bevorzugte organische Moleküle bestehen ausschließlich aus leichten Atomen wie C, H, N, O, F, S, K, Na. Für derartige organische Moleküle, deren elektronische Singulett- und Triplett-Zustände im Wesentlichen aus Übergängen zwischen $\pi$- und $\pi^*$-Molekülorbitalen resultieren, ist - wie bereits erwähnt - die wirksame Spin-Bahn-Kopplung (SBK) so klein, dass die Relaxationsübergänge aus dem $S_1$- in den energetisch tiefer liegenden $T_1$-Zustand (Down-Intersystem-Crossing) und umgekehrt aus dem $T_1$-Zustand in den $S_1$-Zustand (Up-Intersystem-Crossing) stark verboten sind bzw. kaum auftreten.

Erfindungsgemäß wird dieses Verbot aufgehoben: Die organischen Moleküle (Emitter-Moleküle), gemäß der

Formel V, können, z. B. in opto-elektronischen Vorrichtungen, in Matrixmaterialien, z. B. in einer OLED-Emissionsschicht, eindotiert sein. Dieser Matrix werden erfindungsgemäß optisch inerte Atome oder Moleküle (sog. "Zusatzstoffe") zur Reduktion der Inter-System-Crossing Zeitkonstante des organischen Moleküls zugemischt. Diese optisch inerten Atome oder Moleküle zeichnen sich durch eine hohe Spin-Bahn-Kopplung (SBK) aus (SBK-Konstante der Atome oder Molekülbausteine größer 1000 $cm^{-1}$, siehe dazu die weiter unten gegebenen Erläuterungen). Diese Zusatzstoffe werden zum Beispiel in einer Konzentration eingebracht, die etwa der der Emitter-Moleküle entspricht oder höher ist. Die Zusatzstoffe können z. B. auch in doppelt bis zehnfach so hoher Konzentration wie die organischen Emittermoleküle verwendet werden. Allgemein ist das numerische Verhältnis zwischen organischen Emitter-Molekülen und optisch inerten Atomen oder Molekülen 1 : 0,1 bis 1 : 5 oder 1 : 10, bevorzugt 1 : 0,2 bis 1 : 5, besonders bevorzugt 1:1. Damit ist die Verteilungs-Wahrscheinlichkeit gegeben, dass mindestens ein Zusatzteilchen/Zusatzmolekül mit hoher SBK in nächster Nachbarschaft eines EmitterMoleküls liegt. Hierdurch wird eine externe SBK induziert, die den Prozess des Inter-System-Crossings stark beschleunigt, d. h. die Zeitkonstante des Inter-System-Crossings entsprechend verkürzt. Das bewirkt eine schnelle Relaxation vom $S_1$- zum $T_1$-Zustand und ebenso eine schnelle thermische Rückbesetzung gemäß Gleichung (1). Damit wird der Singulett-Harvesting-Effekt für organische Moleküle ermöglicht.

Es ist auch erfindungsgemäß möglich, die Matrix mit geeigneten, die SBK erhöhenden Substitutionen zu verändern; z. B. können die Matrixpolymere chemisch gebundene Br- bzw. J-Atome enthalten, und somit übernimmt die Matrix die Funktion des Zusatzstoffes. Ebenso können die Emittermoleküle z. B. chemisch gebundene Br- bzw. J-Atome oder andere, die SBK erhöhende Substitutionen enthalten. In diesen besonderen Fällen kann unter günstigen Bedingungen die Menge des Zusatzstoffes stark reduziert werden, oder die Zugabe eines Zusatzstoffes kann sogar gänzlich entfallen.

Daher betrifft die vorliegende Erfindung eine Zusammensetzung gemäß Anspruch 1.

Beispiele für die Zusatzstoffe sind:

- Edelgase (besonders bevorzugt):
  Krypton (Kr) aber besonders bevorzugt Xenon (Xe). Diese Gase werden während des Herstellungsprozesses eines optoelektronischen Bauelements in die mit den Emitter-Molekülen dotierte Matrix, die zur Ausbildung der Emissionsschicht verwendet wird, eingeleitet. Dabei ist auf eine Gassättigung bei einem Gasdruck von einer Atmosphäre (1013.25 hPa), gegebenenfalls unter erhöhtem Gasdruck von bis etwa 3 atm (ca. 300 kPa), z. B. von etwa 2 atm (ca. 200 kPa), zu achten. Die Emissionsschicht wird unter dieser Gasatmosphäre zum Beispiel über Spin-Coating oder andere nass-chemische Verfahren aufgebracht.
- Brom- und Jod-haltige Substanzen, wobei Jod-haltige Substanzen besonders bevorzugt sind.
  Der zur Herstellung der Emissionsschicht eines optoelektronischen Bauelements verwendeten Lösung werden Br- oder besonders bevorzugt I-haltige Substanzen zugegeben, zum Beispiel Alkyl-Bromide, Alkyl-Jodide (z. B. Ethyljodid, Propyljodid), Aryl-Bromid, Aryl-Jodid (z. B. Naphthyljodid)
  Opto-elektronische Vorrichtungen unter Verwendung dieser Zusatzstoffe werden nass-chemisch hergestellt.
- Das Matrixmaterial der Emissionsschicht eines optoelektronischen Bauelements kann aus Brom-, aber besonders bevorzugt aus Jod-haltigen Substanzen oder Polymergebundenem Br oder I bestehen oder diese Substanzen aufweisen (z. B. Poly(-4-Jodostyren). Diese Halogene können sich auch in chemisch gebundener Form in den Polymerseitengruppen befinden.
  Opto-elektronische Vorrichtungen unter Verwendung dieser Zusatzstoffe werden nass-chemisch hergestellt.
- Als Zusatzstoffe eignen sich auch Nanoteilchen aus Metallatomen der zweiten oder bevorzugt der dritten Periode der Übergangsmetalle oder Gadolinium. Opto-elektronische Vorrichtungen unter Verwendung dieser Zusatzstoffe werden nass-chemisch oder über Vakuum- oder über Vaporphase-Deposition-Verfahren hergestellt.
- Bevorzugt als Zusatzstoffe sind Gd-Komplexe. Diese können den bei der Herstellung verwendeten Lösungen der Emissionsschichten für nass-chemische Verarbeitungen beigemischt werden oder für den Fall der Herstellung der opto-elektronischen Vorrichtungen mittels Vakuum-Sublimation oder Vaporphase-Deposition co-verdampft werden. Besonders bevorzugt sind chemisch stabile Gd-Komplexe, die in dem für die Anwendung benötigten Spektralbereich optisch inert sind. Beispiele sind: $Gd(Cyclopentadien)_3$, $Gd(Tetramethylheptadien)_3$, Gd-Acetat, $Gd(acac)_3$, $Gd(TMHD)_3$, Gd-2-Ethylhexanoat usw. Gd-Ionen gelten als optisch inert und können in einem weiteren Aspekt der Erfindung eingesetzt werden. Zum Beispiel können diese Gd-Ionen auch mit den organischen Emitter-Molekülen chemische Bindingen eingehen. Zum Beispiel können Gd-Komplexe gebildet werden. Als weitere Zusatzstoffe seien BleiVerbindungen genannt, z. B. $Pb(CH_3COO)_2$.
- Als Zusatzstoffe eignen sich allgemein Atome oder Moleküle oder Nano-Teilchen, die im Emissions- bzw. relevanten HOMO-/LUMO-Bereich des Emitters keine Absorptionen oder Emissionen aufweisen und somit in diesen Bereichen optisch als optisch inert gelten. Die Zusatzstoffe, bzw. deren atomare Bausteine, sollen außerdem eine hohe SBK-Konstante aufweisen, die bevorzugt größer 1000 $cm^{-1}$, besonders bevorzugt größer 3000 $cm^{-1}$ ganz besonders bevorzugt größer 4000 $cm^{-1}$ ist.

## OLED-Devices als optoelektronische Vorrichtungen

In einem weitern Aspekt der Erfindung wird die hier beschrieben Zusammensetzung in einer Emitterschicht in einer optoelektronischen (organisch elektronischen) Vorrichtung, insbesondere einer OLED verwendet.

Die OLED-Devices können nach aus dem Stand der Technik bekannten Verfahren hergestellt werden (vergleiche H. Yersin, "Highly Efficient OLEDs with Phosphorescent Materials", Wiley-VCH, Weinheim, Germany 2008).

Bei einer bevorzugten Ausgestaltung einer organischen Leuchtidiode (OLED) beträgt der Anteil der Zusammensetzung (organischer Emitter und Zusatzstoff) in der Emitterschicht zwischen 2 Gew.-% und 100 Gew.-%, bevorzugt zwischen 6 Gew.-% und 30 Gew.-%.

## Weitere optoelektronische Vorrichtungen

Ein anderer Aspekt der Erfindung ist die Verwendung der erfindungsgemäßen Zusammensetzung aus organischem Molekül und optisch inertem Atom oder optisch inertem Molekül zum Einsatz in Licht emittierenden elektrochemischen Zellen (light emitting electrochemical cells, LEECs), OLED-Sensoren (z. B. OLED-Sauerstoffsensoren), insbesondere in einem nicht hermetisch nach außen abgeschirmten Gas- und Dampf-Sensor, optischen Temperatur-Sensoren, organischen Solarzellen (organic solar cells, OSCs; organic photovoltaics, OPVs), organischen Feldeffekttransistoren, organischen Dioden, organischen Photodioden und "down conversion" Systemen.

Generell kann der Anteil der Zusammensetzug in einer Emitterschicht einer optoelektronischen Vorrichtung 2 bis 100 Gew.-%, bevorzugt 6 bis 30 Gew.-%, bezogen auf das Gesamtgewicht der Emitterschicht, betragen.

In einem weiteren Aspekt betrifft die Beschreibung ein Verfahren zur Verringerung der (radiativen) Emissionslebensdauer und ein Verfahren zur Erhöhung der Elektrolumineszenz-Quantenaubeute eines organischen Moleküls als Emitter in einer optoelektronischen Vorrichtung. Dabei wird ein organisches Molekül, das in einen $\Delta E(S_1\text{-}T_1)$-Wert zwischen dem untersten angeregten Singulett- ($S_1$) und dem darunter liegenden Triplett-Zustand ($T_1$) von kleiner als 3000 $cm^{-1}$ (bevorzugt von kleiner als 2500 $cm^{-1}$) aufweist, in die Nähe eines optisch inerten Atoms oder Moleküls verbracht (gegebenenfalls über eine chemische Bindung), so dass das organische Molekül mit dem optisch inerten Atoms oder Molekül in Wechselwirkung treten kann. Aufgrund einer Spin-Bahn-Kopplungs-Konstanten von größer 1000 $cm^{-1}$ des optisch inerten Atoms oder Moleküls oder von Teilen des optisch inerten Moleküls wird eine kurze mittlere Emissionslebensdauer (aus dem Singulett $S_1$- und dem Triplett $T_1$-Zustand) des organischen Moleküls sowie eine Erhöhung der Emissions-Quantenaubeute erreicht.

Die Beschreibung betrifft weiterhin ein Verfahren zur Umwandlung der bei der Elektrolumineszenz generierten Triplett-Anregungsenergie eines organischen Moleküls in Fluoreszenzenergie. Dabei wird ein organisches Molekül, das einen $\Delta E(S_1\text{-}T_1)$-Wert zwischen dem untersten angeregten Singulett- ($S_1$) und dem darunter liegenden Triplett-Zustand ($T_1$) von kleiner als 3000 $cm^{-1}$ (bevorzugt kleiner als 2500 $cm^{-1}$) aufweist, derart mit einem optisch inerten Atom oder Molekül in Wechselwirkung gebracht, dass eine Triplett-Anregungsenergie des organischen Moleküls über einen Singulettzustand des organischen Moleküls durch thermische Aktivierung bei einer Temperatur, oberhalb der zum Beispiel OLEDs eingesetzt werden, d. h. zum Beispiel oberhalb von -30 °C, in Fluoreszenzenergie umgewandelt wird.

Die Beschreibung betrifft auch ein Verfahren zur Auswahl von organischen Molekülen, deren $\Delta E(S_1\text{-}T_1)$-Wert zwischen dem untersten angeregten Singulett- ($S_1$) und dem darunter liegenden Triplett-Zustand ($T_1$) kleiner als 3000 $cm^{-1}$, bevorzugt kleiner als 2500 $cm^{-1}$ oder 1500 $cm^{-1}$, besonders bevorzugt kleiner als 1000 $cm^{-1}$ ist. Das Verfahren umfasst mindestens zwei Schritte, nämlich: erstens die Bestimmung des $\Delta E(S_1\text{-}T_1)$-Wertes organischer Moleküle mittels a) einer quantenmechanischen Molekülrechnung, b) Messung der Temperaturabhängigkeit der Fluoreszenz- und der Phosphoreszenz-Intensität, oder c) der Messung der Temperaturabhängigkeit der Emissionsabklingzeit, und
zweitens die Ermittlung der von organischen Molekülen, deren $\Delta E(S_1\text{-}T_1)$-Wert kleiner als 3000 $cm^{-1}$, bevorzugt kleiner als 2500 $cm^{-1}$ oder 1500 $cm^{-1}$, besonders bevorzugt kleiner als 1000 $cm^{-1}$ ist. Die so ermittelten organischen Moleküle, weisen einen $\Delta E(S_1\text{-}T_1)$-Wert zwischen dem untersten angeregten Singulett- ($S_1$) und dem darunter liegenden Triplett-Zustand ($T_1$) von kleiner als 3000 $cm^{-1}$, bevorzugt kleiner als 2500 $cm^{-1}$ oder 1500 $cm^{-1}$, besonders bevorzugt kleiner als 1000 $cm^{-1}$ auf.

## Beispiele

Aus der Vielzahl der realisierbaren organischen Moleküle mit kleiner Singulett $S_1$-Triplett $T_1$-Energiedifferenz seien am Beispiel der Emitter gemäß den Formeln I bis III einige Beispiele aufgeführt, die folgende Eigenschaften aufweisen:

- Die Materialien stellen sehr gute Emitter dar.
- Die Absorptions- und Fluoreszenz-Übergänge zwischen den Zuständen $S_0$ und $S_1$ sind erlaubt. Damit sind die Emissionsabklingzeiten $\tau(S_1)$ kurz.

- Die Beispiele umfassen Moleküle mit Emissionen aus einem weiten Spektralbereich.

Ein Beispiel für ein organisches Molekül ist durch Formel IV definiert.

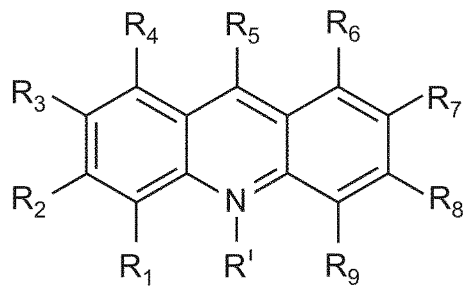

Formel IV

Hierin sind $R_1$ bis $R_9$ = H, Br, I und/oder Gruppen, die in den Beispielen für die oben genannten Donatoren D und/oder Akzeptoren A zusammengefasst sind. Benachbarte $R_i$, $R_j$ und $R_k$ aus $R_1$ bis $R_9$ können konjugierte aromatische oder heteroaromatische Ringe sein. Bevorzugt bewirken Br und/oder I auch eine Erhöhung der Spin-Bahn-Kopplung.

R' ist entweder nicht vorhanden oder H, Alkyl, O, S.

Das erfindungsgemäße organische Molekül ist durch Formel V definiert.

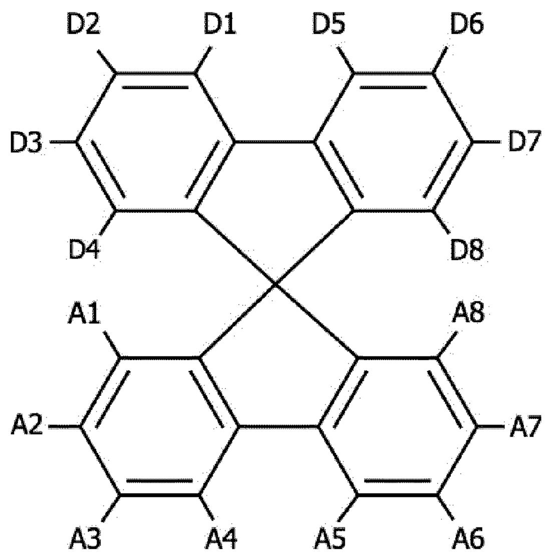

Formel V

Hierin sind D1 bis D8 unabhängig voneinander H, Br, I und/oder Donatorgruppen wie oben durch D definiert. Die Donatorgruppen können gleich oder verschieden sein. Dabei muss mindestens eine Donatorgruppe vorhanden sein. A1 bis A8 sind unabhängig voneinander H, Br, I und/oder Akzeptorgruppen wie oben durch A definiert. Dabei muss mindestens eine Akzeptorgruppe vorhanden sein. Die Akzeptorgruppen können gleich oder verschieden sein.

Für Moleküle nach den Formeln IV und V ist es besonders wichtig, dass eine Auswahl so getroffen wird, dass innerhalb des resultierenden Moleküls eine Charge-Transfer-Übergangskomponente auftritt. Diese lässt sich nach dem Stand der Technik durch DFT- bzw. TDDFT-Rechnungen ermitteln. Br oder I kann auch über eine kurze Alkylgruppe (mit C = 1 oder 2) an das aromatische Grundgerüst gebunden sein. Durch chemisch gebundenes Br oder I kann die intra-molekulare Spin-Bahn-Kopplung auch in gewünschter Weise erhöht werden. In diesem Fall könnte die Menge eines inerten Zusatzstoffes zur Erhöhung der Spin-Bahn-Kopplung stark reduziert werden oder gänzlich entfallen.

Für den Fall, dass der Zusatzstoff kovalent mit dem organischen Molekül verbunden ist, betrifft die erfindungsgemäße Zusammensetzung somit nur ein Molekül. Der in diesem Fall kovalent an das organische Molekül gebundene Zusatzstoff ist dann für eine Erhöhung der Spin-Bahn-Kopplung verantwortlich. Bevorzugte derartige Zusatzstoffe sind Iod und/oder Brom.

In einem weiteren Aspekt betrifft die Beschreibung die Verwendung einer Mischung (Zusammensetzung) aufweisend oder bestehend aus Glukose und/oder Trehalose als Matrix mit einer Zusammensetzung gemäß der hier beschriebenen Art in einer optoelektronischen Vorrichtung. Das Verhältnis von Glukose zu Trehalose kann von 5:1 bis 1:5 betragen. Bevorzugt ist ein Verhältnis von 1:1.

In den Figuren 3 bis 13 sind konkrete Ausführungsbeispiele, sowie zeitintegrierte und zeitaufgelöste Spektren und Abklingkurven sowie für ein Beispiel die errechneten HOMO- und LUMO-Konturkurven zusammengefasst.

**Figuren**

Figur 1: Prinzipieller Aufbau eines OLEDs. Die Abbildung ist nicht maßstabsgerecht gezeichnet.

Figur 2: Erläuterungen des Elektro-Lumineszenz-Verhaltens a für typische organische Moleküle nach dem Stand der Technik und b für erfindungsgemäß selektierte Moleküle, die in ihrer unmittelbaren Umgebung durch Zusatzstoffe modifiziert sind, um das "Singulett-Harvesting-Verfahren für organische Moleküle" zu ermöglichen.

Figur 3: Chemische Struktur von Acridin Gelb als Beispiel für ein rein organisches Emittermolekül, das unter Verwendung der Kombination von Emittermolekül und Zusatzstoff zur Ausnutzung des Singulett-Harvesting-Effektes geeignet ist.

Figur 4: Zeitintegriertes Emissionsspekrum von Acridin Gelb als organisches Molekül (ohne Zusatzstoff) gelöst in einer Glukose-Trehalose Matrix in einer Konzentration von 1,67 μMol pro 1 g der Mischung Glukose-Trehalose gemessen bei 300 K und angeregt bei 378 nm. Die Emission mit dem Maximum bei 508 nm repräsentiert weitgehend eine Überlagerung der spontanen und der verzögerten Fluoreszenz aus dem $S_1$-Zustand. Verwendet wurde eine 1 : 1 Glukose/Trehalose-Mischung. Trehalose ist ein Zweifachzucker mit der Summenformel $C_{12}H_{22}O_{11}$. Die Emissionsquantenausbeute der Gesamtemission bestehend aus der spontanen und der verzögerten Fluoreszenz sowie der Phosphoreszenz beträgt (95 $\pm$ 5) %. Die Emissionsquantenausbeute wurde mittels eines kommerziellen Messgerätes bestimmt.

Figur 5: Zeitverzögertes Emissionsspekrum von Acridin Gelb (ohne Zusatzstoff) gelöst in einer Glukose-Trehalose Matrix in einer Konzentration von 1,67 μMol pro 1 g der Mischung Glukose-Trehalose gemessen bei 300 K und angeregt bei 378 nm. Das Spektrum wurde nach einer Zeitverzögerung von t = 100 ms mit einem Zeitfenster von $\Delta t$ = 1000 ms registriert. Hierdurch wird die langlebige Phosphoreszenzbande, die aus dem $T_1$-Zustand resultiert und bei 570 nm liegt, deutlich hervorgehoben. Die (zeitverzögerte) Haupt-Emissionsbande bei 508 nm repräsentiert die verzögerte Fluoreszenz aus dem $S_1$-Zustand.

Figur 6: Zeitverzögertes Emissionsspekrum von Acridin Gelb (ohne Zusatzstoff) gelöst in einer Glukose-Trehalose Matrix in einer Konzentration von 1,67 μMol pro 1 g der Mischung Glukose-Trehalose gemessen bei 77 K und angeregt bei 378 nm. Das Spektrum wurde nach einer Zeitverzögerung von t = 100 ms und mit einem Zeitfenster von $\Delta t$ = 1000 ms registriert. Bei dieser tiefen Temperatur ist eine thermische Rückbesetzung des Singulett-Zustandes $S_1$ nicht gegeben. Ferner wird durch die gewählte Zeitverzögerung die Registrierung der kurzlebigen (wenige ns) spontanen Fluoreszenz verhindert. Es handelt sich also um eine Phosphoreszenz mit dem Maximum bei 570 nm, die aus dem $T_1$-Zustand resultiert. Infolgedessen repräsentiert auch die beobachtete Schulter bei der Wellenlänge 570 nm in dem in Figur 5 gezeigten Spektrum eine Phosphoreszenz.

Die Registrierung von zeitverzögerten Emissionsspektren von Acridin Gelb mit Zusatzstoff $Pb(CH_3COO)_2$ zeigt bei 300 K keine nachweisbare Phosphoreszenzbande aus dem $T_1$-Zustand, sondern nur eine verzögerte Fluoreszenzbande aus dem $S_1$-Zustand. Die Substanz wurde bei 378 nm angeregt, und das Spektrum wurde nach einer Zeitverzögerung von t = 100 ms mit einem Zeitfenster von $\Delta t$ = 1000 ms registriert. Als Matrix wurde eine Glukose-Trehalose-Mischung verwendet. Diese Matrix enthielt 2,50 μMol (Acridin Gelb) und als Zusatzstoff 25,3 μMol ($Pb(CH_3COO)_2$) pro 1 g der Mischung Glukose-Trehalose. Das entspricht einem (Acridin Gelb)/($Pb(CH_3COO)_2$)-Molverhältnis von etwa 1 : 10. Dieses Ergebnis demonstriert deutlich, dass der Up-Inter-System-Crossing-Effekt effektiv ist und damit das Auftreten der Phosphoreszenz aus dem $T_1$-Zustand unterbindet. Folglich ist gezeigt, dass die Anregungsenergie, die in den

$T_1$-Zustand gelangt, bei T = 300 K durch die thermische Aktivierung über den $S_1$-Zustand, d. h. als Fluoreszenz, emittiert wird. Somit ist die Eignung dieser Kombination (Emittermolekül (organisches Molekül) und Zusatzstoff) zur Ausnutzung des Singulett-Harvesting-Effektes gegeben.

Figur 7: Chemische Struktur von Acridin Orange als weiteres Beispiel für ein rein organisches Emittermolekül, das unter Verwendung der Kombination von Emittermolekül und Zusatzstoff zur Ausnutzung des Singulett-Harvesting-Effektes geeignet ist.

Figur 8: Zeitverzögertes Emissionsspekrum von Acridin Orange (ohne Zusatzstoff) gelöst in einer Glukose-Trehalose Matrix in einer Konzentration von 1,86 $\mu$Mol pro 1 g der Mischung Glukose-Trehalose gemessen bei 300 K und angeregt bei 378 nm. Das Spektrum wurde nach einer Zeitverzögerung von t = 20 ms mit einem Zeitfenster von $\Delta$t = 500 ms registriert. Die Emission mit dem Maximum bei 530 nm repräsentiert ausschließlich eine verzögerte Fluoreszenz aus dem $S_1$-Zustand, weil die kurzlebige (einige ns) spontane Fluoreszenz nach der Zeitverzögerung bereits abgeklungen ist. Verwendet wurde eine 1 : 1 Glukose-Trehalose-Mischung.

Figur 9: Zeitverzögertes Emissionsspekrum von Acridin Orange (ohne Zusatzstoff) gelöst in einer Glukose-Trehalose Matrix in einer Konzentration von 1,86 $\mu$Mol pro 1 g der Mischung Glukose-Trehalose gemessen bei 77 K und angeregt bei 378 nm. Das Spektrum wurde nach einer Zeitverzögerung von t = 20 ms mit einem Zeitfenster von $\Delta$t = 1000 ms registriert. Bei dieser tiefen Temperatur ist eine thermische Rückbesetzung des Singulett-Zustandes $S_1$ nicht gegeben, d. h. die beobachtete Emission mit dem Maximum bei 603 nm stellt keine verzögerte Fluoreszenz dar. Ferner wird durch die gewählte Zeitverzögerung die Registrierung der kurzlebigen (wenige ns) spontanen Fluoreszenz verhindert. Infolgedessen repräsentiert das beobachtete Spektrum eine Phosphoreszenz $T_1$-Zustand.

Die Untersuchungen mit Acridin Orange zeigen eindeutig, dass eine verzögerte Fluoreszenz auftritt. Damit ist gezeigt, dass bei T = 300 K die Anregungsenergie, die in den $T_1$-Zustand gelangt, durch die thermische Aktivierung über den $S_1$-Zustand, d. h. als Fluoreszenz, emittiert wird. Somit ist die Eignung bei Kombination von derartigen Emittermolekülen und Zusatzstoffen zur Ausnutzung des Singulett-Harvesting-Effektes gegeben.

Figur 10: Chemische Strukturen von weiteren Acridin-Derivaten als Beispiele für rein organische Emittermoleküle, die unter Verwendung der Kombinationen von Emittermolekülen und Zusatzstoffen zur Ausnutzung des Singulett-Harvesting-Effektes geeignet sind.

In einer möglichen Ausführungsform der Zusammensetzung ist das organische Molekül mit dem die Spin-Bahn-Kopplung erhöhenden Atom oder mit dem die Spin-Bahn-Kopplung erhöhenden Molekül kovalent verbunden, wie hier beispielhaft durch die erste Verbindung in der dritten Zeile gezeigt ist. Analog zu den in der letzten Zeile gezeigten Beispiel-Molekülen sind weitere organische Moleküle bevorzugt geeignet, und zwar organische Emittermoleküle, die an den Positionen $R_2$, $R_3$, $R_7$ und/oder $R_8$ gemäß Formel IV Aldehydgruppen aufweisen.

Organische Emitter-Moleküle können auch geladene organische Moleküle sein, und es können Gegenionen vorhanden sein. Diese Emittermoleküle können dann bevorzugt in licht-emittierenden elektrochemischen Zellen (LEECs oder LECs) eingesetzt werden, deren prinzipieller Aufbau dem Fachmann bekannt ist. Bei der Verwendung dieser geladenen organischen Moleküle in OLEDs ist es gegebenenfalls empfehlenswert, die kleinen Gegenionen durch größere, gleichermaßen geladene Gegenionen, wie $(PF_6)^-$, $(BF_4)^-$, $[CF_3SO_2]^-$, einfach negativ geladenes Hexaphenylphosphat, einfach negativ geladenes Tetraphenyborat usw. zu ersetzen.

Die Gegenionen für positiv geladene organische Emittermoleküle können in einer bevorzugten Ausführungsform die Funktion der Zusatzstoffe erfüllen. Beispiele sind $Br^-$, $J^-$, $(AsF_6)^-$, $(SbF_6)^-$, einfach negativ geladenes Hexaphenylarsenat, einfach negativ geladenes Hexa-phenylantimonat. Die Gegenionen für negativ geladene organische Emittermoleküle können z. B. $Rb^+$, $Cs^+$ und/oder $Ba^+$ sein.

Figur 11: Chemische Struktur einer Spiro-Verbindung als Beispiel zur Formel V sowie HOMO- und LUMO-Kontur-Darstellungen (durch DFT-Rechnungen ermittelt). Letztere zeigen, dass bei einem elektronischen HOMO-LUMO-Übergang ein ausgeprägter Charge-transfer erfolgt und dass infolgedessen ein kleiner $\Delta E(S_1-T_1)$-Wert erwartet werden kann.

Figur 12: Emissionsabklingkurven bei T = 300 K für die unter Figur 11 dargestellte Spiro-Verbindung. Die Anregung der Emission erfolgte mit einem Laserpuls mit einer Pulsbreite von 70 ps bei 378 nm. Die Proben waren entgast, um den Luftsauerstoff zu entfernen. (a) Emitter-Molekül gelöst in (flüssigem) Toluol ($c \approx 10^{-5}$ Mol/l). Die Messung zeigt deutlich, dass zwei Abklingkomponenten auftreten. Die kurzlebige Komponente von $\approx$ 40 ns entspricht einer spontanen Fluoreszenz, während die langlebige Komponente von $\approx$ 5 $\mu$s der thermisch aktivierten verzögerten Fluoreszenz zugeordnet wird. Letzteres kann dadurch belegt werden, dass beide Emissionskomponenten spektral nicht getrennt auftreten, sondern an der Position der spontanen Fluoreszenz mit dem Maximum bei etwa 500 nm liegen. Bei Raumtemperatur wurde keine Phosphoreszenz beobachtet, bei tiefer Temperatur (z.B. bei T = 10 K) lässt sich diese dagegen gut nachweisen. Die entsprechende Abklingzeit liegt im Sekundenbereich. Aus der Energiedifferenz zwischen dem Emissionsmaximum des Phosphoreszenz- und des Fluoreszenzspektrektrums (z.B. bei T = 10 K) lässt sich der $\Delta E(S_1-T_1)$-Wert zu ungefähr 800 $cm^{-1}$ abschätzen. (b) Emissionsabklingverhalten der Spiro-Verbindung gelöst in einer Polystyren-Matrix (= Poly(1-phenylethan-1,2-diyl)) bzw. in einer Poly(4-Iodostyren)-Matrix (genauer: Poly(1-(4-iodophenyl)ethan-1,2-diyl) mit einer Konzentration von $c \approx 1$ Gewichts-%. Die Abklingzeit der thermisch aktivierten Fluoreszenz

ist für diese Matrizen länger als in (a), weil sich der Emitter in einer festen Matrix Umgebung befindet. Das Abklingen der Emission ist hier nur für den relativ langen Millisekunden-Zeitbereich wiedergegeben, d. h. für einen Zeitbereich, in dem die thermisch aktivierte Fluoreszenz, aber keine spontane Fluoreszenz auftritt. Die Iodierung der Matrix (Erhöhung der externen Spin-Bahn-Kopplung) führt zu einer deutlichen Erhöhung der Intersystem-Crossing-Rate und damit zu einer signifikanten Verkürzung der Emissionslebensdauer der thermisch aktivierten Komponente. Somit repräsentiert diese organische Emittersubstanz (Spiro-Verbindung) bei Verwendung eines Zusatzstoffes (jodierte Marix) eine für das Singulett-Harvesting-Verfahren geeignete Kombination.

Figur 13: Chemische Strukturen weiterer Spiro-Verbindungen. Die obere Verbindung zeigt bei Raumtemperatur und dotiert in PMMA mit c ≈ 1 Gewichts-% eine spontane und eine thermisch aktivierte Fluoreszenz mit einem Emissionsmaximum bei ≈ 530 nm. Die Emissionsquantenausbeute beider Komponenten beträgt (50 ± 10) %. Die Verbindungen mit den darunter dargestellten Strukturformeln zeigen ebenfalls thermisch aktivierte Fluoreszenz.

**Patentansprüche**

1. Zusammensetzung, aufweisend

- ein organisches Molekül zur Emission von Licht, das einen $\Delta E(S_1\text{-}T_1)$-Wert zwischen dem untersten angeregten Singulett- ($S_1$) und dem darunter liegenden Triplett-Zustand ($T_1$) von kleiner als 3000 $cm^{-1}$ aufweist und
- ein Atom oder ein Molekül zur Reduktion der Inter-System-Crossing Zeitkonstante des organischen Moleküls auf kleiner als 300 ms,

wobei das organische Molekül eine Struktur der Formel V aufweist

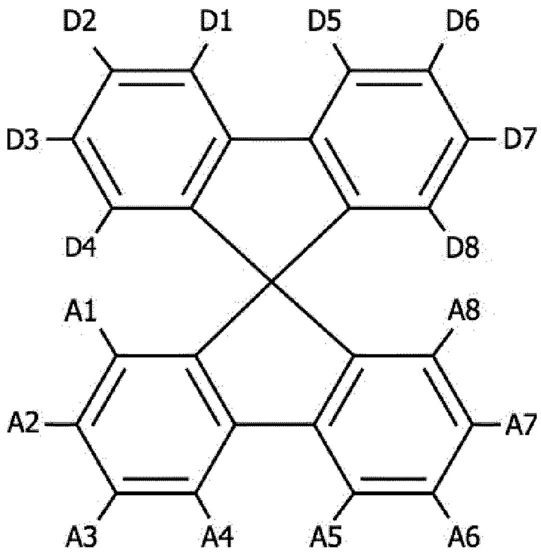

Formel V

wobei

D1 bis D8 unabhängig voneinander H, Br, I und/oder Gruppen sind, die einen Donator D darstellen;
die Donatorgruppen gleich oder verschieden sein können;
mindestens eine Donatorgruppe vorhanden ist;
wobei benachbarte Di, Dj und Dk aus D1 bis D8 optional konjugierte aromatische oder heteroaromatische Ringe sind, wobei diese optional Donator-Funktion aufweisen;
A1 bis A8 unabhängig voneinander H, Br, I und/oder Gruppen sind, die einen Akzeptor A darstellen;
wobei benachbarte Ai, Aj und Ak aus A1 bis A8 optional konjugierte aromatische oder heteroaromatische Ringe sind, wobei diese optional Akzeptor-Funktion aufweisen;
wobei die Akzeptorgruppen gleich oder verschieden sind; und
mindestens eine Akzeptorgruppe vorhanden ist; und

wobei Br und/oder I optional über eine Alkylgruppe (mit C = 1 oder 2) an das organische Molekül der Formel V gebunden ist;
wobei
das zur Reduktion der Inter-System-Crossing Zeitkonstante des Emittermoleküls verwendete zusätzliche Atom oder Molekül ausgewählt ist aus der Gruppe bestehend aus: Krypton- und Xenon-Edelgasen, Brom-aufweisenden Substanzen, Jod-aufweisenden Substanzen, Metallatomen, Metall-Nanoteilchen, Metallionen, Gd-Komplexen und Bleikomplexen.

**2.** Die Zusammensetzung nach Anspruch 1, wobei das zur Reduktion der Inter-System-Crossing Zeitkonstante des Emittermoleküls verwendete zusätzliche Atom oder Molekül oder Teile davon eine Spin-Bahn-Kopplungs-Konstante von größer 1000 $cm^{-1}$, bevorzugt von größer 3000 $cm^{-1}$, besonders bevorzugt von größer 4000 $cm^{-1}$ aufweist.

**3.** Die Zusammensetzung nach Anspruch 1 oder 2, wobei das organische Molekül in der Zusammensetzung einen $\Delta E(S_1\text{-}T_1)$-Wert zwischen dem untersten angeregten Singulett- und dem darunter liegenden Triplett-Zustand von kleiner als 2500 $cm^{-1}$, bevorzugt von kleiner als 1500 $cm^{-1}$, besonders bevorzugt von kleiner als 1000 $cm^{-1}$ aufweist.

**4.** Zusammensetzung nach Anspruch 1 bis 3, wobei
das zur Reduktion der Inter-System-Crossing Zeitkonstante des Emittermoleküls verwendete zusätzliche Atom oder Molekül im Emissionsbereich und/oder im HOMO-/LUMO-Bereich des organischen Moleküls keine Absorptionen oder Emissionen aufweist.

**5.** Zusammensetzung nach Anspruch 1 bis 4, wobei das numerische Verhältnis zwischen den zur Reduktion der Inter-System-Crossing Zeitkonstante des Emittermoleküls verwendete Atom oder Molekül und den Emittermolekülen 1 : 0,1 bis 1 : 50 beträgt.

**6.** Zusammensetzung nach Anspruch 1 bis 5, wobei das Atom oder Molekül zur Reduktion der Inter-System-Crossing Zeitkonstante des organisches Moleküls kovalent mit dem organischen Molekül verbunden ist, und das kovalent an das organische Molekül gebundene Atom oder Molekül zur Reduktion der Inter-System-Crossing Zeitkonstante eine Erhöhung der Spin-Bahn-Kopplung bewirkt.

**7.** Zusammensetzung nach Anspruch 6, wobei das kovalent mit dem organischen Molekül verbundene Atom oder Molekül zur Reduktion der Inter-System-Crossing Zeitkonstante des organisches Moleküls Iod und/oder Brom ist.

**8.** Zusammensetzung nach Anspruch 1 bis 7, wobei das organische Molekül mindestens ein Deuterium-Atom aufweist.

**9.** Zusammensetzung nach Anspruch 1 bis 7, wobei das organische Molekül in eine Matrix eingelagert oder mit ihr vernetzt ist, wobei die Matrix optional mindestens ein Deuterium-Atom aufweist.

**10.** Zusammensetzung nach Anspruch 9, wobei die Matrix eine Polymer-Matrix oder eine polymer-vernetzte Matrix ist, wobei die Matrix mit dem organischen Molekül kovalent verknüpft ist.

**11.** Zusammensetzung nach Anspruch 9 oder 10, wobei die Matrix mindestens einen die Spin-Bahn-Kopplung erhöhenden und damit die Intersystem-Crossing-Zeitkonstante erniedrigenden kovalent gebundenen Zusatzstoff aufweist, insbesondere ein Br-Atom oder ein J-Atom.

**12.** Zusammensetzung aufweisend

- eine Matrix, aufweisend oder bestehend aus Glukose und/oder Trehalose und
- eine Zusammensetzung gemäß Anspruch 1 bis 8,

insbesondere zur Verwendung in einer optoelektronischen Vorrichtung.

**13.** Verwendung einer Zusammensetzung nach Anspruch 1 bis 9 in einer Emitterschicht in einer optoelektronischen Vorrichtung.

**14.** Verfahren zur Herstellung einer optoelektronischen Vorrichtung, wobei eine Zusammensetzung nach Anspruch 1 bis 12 verwendet wird.

**15.** Optoelektronische Vorrichtung, aufweisend eine Zusammensetzung nach den Ansprüchen 1 bis 12.

**16.** Optoelektronische Vorrichtung nach Anspruch 15, wobei der Anteil der Zusammensetzung in einer Emitterschicht 0,5 % bis 100 Gew.-%, bevorzugt 6 bis 30 Gew.-%, bezogen auf das Gesamtgewicht der Emitterschicht, beträgt.

**17.** Optoelektronische Vorrichtung nach Anspruch 15 oder 16 in Form einer organischen Leuchtidiode (OLED), **gekennzeichnet durch** eine Zusammensetzung nach Anspruch 1 bis 12 aufweisende Emitterschicht, wobei der Anteil der Zusammensetzung in der Emitterschicht zwischen 0,5 % bis 100 Gew.-%, bevorzugt 6 bis 30 Gew.-%, bezogen auf das Gesamtgewicht der Emitterschicht, beträgt.

**18.** Verwendung nach Anspruch 13, wobei die optoelektronische Vorrichtung ausgewählt ist aus der Gruppe bestehend aus organischen Leuchtdioden (OLEDs), lichtemittierenden elektrochemischen Zellen (LEECs oder LECs), OLED-Sensoren, insbesondere nicht hermetisch nach außen abgeschirmten Gas- und Dampf-Sensoren, optischen Temperatur-Sensoren, organischen Solarzellen (OSCs), organischen Feldeffekttransistoren, organischen Dioden, organischen Photodioden und "down conversion" Systemen.

**19.** Verfahren nach Anspruch 14, wobei die optoelektronische Vorrichtung ausgewählt ist aus der Gruppe bestehend aus organischen Leuchtdioden (OLEDs), lichtemittierenden elektrochemischen Zellen (LEECs oder LECs), OLED-Sensoren, insbesondere nicht hermetisch nach außen abgeschirmten Gas- und Dampf-Sensoren, optischen Temperatur-Sensoren, organischen Solarzellen (OSCs), organischen Feldeffekttransistoren, organischen Dioden, organischen Photodioden und "down conversion" Systemen.

**20.** Optoelektronische Vorrichtung nach Anspruch 15 bis 17, wobei die optoelektronische Vorrichtung ausgewählt ist aus der Gruppe bestehend aus organischen Leuchtdioden (OLEDs), lichtemittierenden elektrochemischen Zellen (LEECs oder LECs), OLED-Sensoren, insbesondere nicht hermetisch nach außen abgeschirmten Gas- und Dampf-Sensoren, optischen Temperatur-Sensoren, organischen Solarzellen (OSCs), organischen Feldeffekttransistoren, organischen Dioden, organischen Photodioden und "down conversion" Systemen.

## Claims

**1.** Composition, comprising

- an organic molecule for emitting light, which has a $\Delta E(S_1\text{-}T_1)$ value between the lowest excited singlet state ($S_1$) and the triplet state ($T_1$) below it of less than 3000 $cm^{-1}$ and
- an atom or a molecule for reducing the intersystem crossing time constant of the organic molecule to less than 300 ms,

wherein the organic molecule comprises a structure of formula V

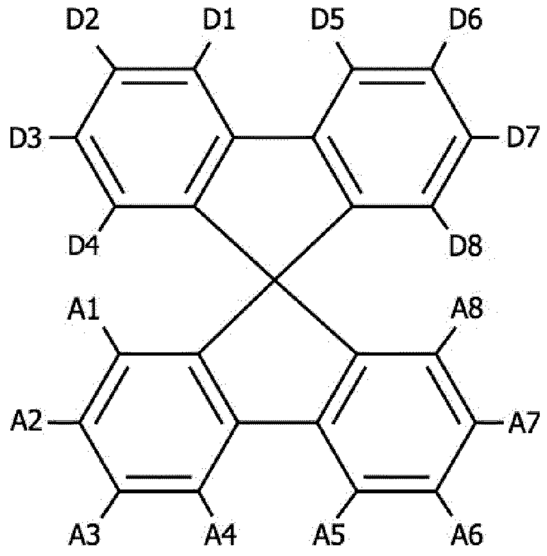

Formula V

wherein

D1 to D8 are independently from each other H, Br, I and/or groups, which represent a donor D;
the donor groups may be the same or different;
at least one donor group is present;
wherein adjacent Di, Dj and Dk from D1 to D8 are optionally conjugated aromatic or heteroaromatic rings, optionally comprising donor function;
A1 to A8 are independently from each other H, Br, I and/or groups, which represent an acceptor A;
wherein adjacent Ai, Aj and Ak from A1 to A8 are optionally conjugated aromatic or heteroaromatic rings, optionally comprising acceptor function;
wherein the acceptor groups may be the same or different; and
at least one acceptor group is present; and
wherein Br and/or I is optionally bound to the organic molecule of formula V via an alkyl group (with C = 1 or 2), wherein
the additional atom or molecule used for reducing the intersystem crossing time constant of the organic emitter molecule is selected from the group consisting of krypton and xenon noble gases, bromine-comprising substances, iodine-comprising substances, metal atoms, metal nanoparticles, metal ions, Gd-complexes and lead complexes.

**2.** The composition according to claim 1, wherein the additional atom or molecule used for reducing the intersystem crossing time constant of the emitter molecule or parts thereof have a spin-orbit coupling constant of greater than 1000 $cm^{-1}$, preferably of greater than 3000 $cm^{-1}$, particularly preferably of greater than 4000 $cm^{-1}$.

**3.** The composition according to claim 1 or 2, wherein the organic molecule in the composition has a $\Delta E(S_1-T_1)$ value between the lowest excited singlet state and the triplet state below it of less than 2500 $cm^{-1}$, preferably of less than 1500 $cm^{-1}$, particularly preferably of less than 1000 $cm^{-1}$.

**4.** The composition according to claims 1 to 3, wherein
the additional atom or molecule used for reducing the intersystem crossing time constant of the emitter molecule does not show absorptions or emissions in the emission region and/or in the HOMO/LUMO region of the organic molecule.

**5.** The composition according to claims 1 to 4, wherein the numerical ratio between the atom or molecule used for reducing the intersystem crossing time constant of the emitter molecule and the emitter molecules is 1:0.1 to 1:50.

**6.** The composition according to claims 1 to 5, wherein the atom or molecule for the reduction of the intersystem

crossing time constant of the organic molecule is covalently bound to the organic molecule, and the atom or molecule for reducing the intersystem crossing time constant of the organic molecule covalently bound to the organic molecule causes an increase of the spin-orbit coupling.

**7.** The composition according to claim 6, wherein the atom or molecule covalently bound to the organic molecule for reducing the intersystem crossing time constant of the organic molecule is iodine and/or bromine.

**8.** The composition according to claims 1 to 7, wherein the organic molecule comprises at least one deuterium atom.

**9.** The composition according to claims 1 to 7, wherein the organic molecule is embedded into a matrix or cross-linked with it, wherein, optionally, the matrix comprises at least one deuterium atom.

**10.** The composition according to claim 9, wherein the matrix is a polymer matrix or a polymer-cross-linked matrix, wherein the matrix is covalently linked to the organic molecule.

**11.** The composition according to claim 9 or 10, wherein the matrix comprises at least one covalently bound additive, in particular a Br atom or a I atom, which increases the spin-orbit coupling and thus decreases the intersystem crossing time constant.

**12.** Composition comprising

- A matrix, comprising or consisting of glucose and/or trehalose, and
- A composition according to claims 1 to 8,

in particular for use in an optoelectronic device.

**13.** Use of a composition according to claims 1 to 9 in an emitter layer of an optoelectronic device.

**14.** Method for producing an optoelectronic device, wherein a composition according to claims 1 to 12 is used.

**15.** Optoelectronic device comprising a composition according to claims 1 to 12

**16.** Optoelectronic device according to claim 15,
wherein the ratio of the composition in an emitter layer is 0.5 % to 100 wt%, preferably 6 to 30 wt%, based on the total weight of the emitter layer.

**17.** Optoelectronic device according to claim 15 or 16 in the form of an organic light-emitting diode (OLED), **characterized by** an emitter layer comprising a composition according to claims 1 to 12, wherein the proportion of the composition in the emitter layer is between 0.5 % and 100 wt%, preferably 6 to 30 wt%, based on the total weight of the emitter layer.

**18.** Use according to claim 13, wherein the optoelectronic device is selected from the group consisting of organic light-emitting diodes (OLEDs), light-emitting electrochemical cells (LEECs or LECs), OLED sensors, in particular gas and vapor sensor not hermetically sealed from the outside, optical temperature sensors, organic solar cells (OSCs), organic field-effect transistors, organic diodes, organic photodiodes and down conversion systems.

**19.** Method according to claim 14, wherein the optoelectronic device is selected from the group consisting of organic light-emitting diodes (OLEDs), light-emitting electrochemical cells (LEECs or LECs), OLED sensors, in particular gas and vapor sensor not hermetically sealed from the outside, optical temperature sensors, organic solar cells (OSCs), organic field-effect transistors, organic diodes, organic photodiodes and down conversion systems.

**20.** Optoelectronic device according to claims 15 to 17, wherein the optoelectronic device is selected from the group consisting of organic light-emitting diodes (OLEDs), light-emitting electrochemical cells (LEECs or LECs), OLED sensors, in particular gas and vapor sensor not hermetically sealed from the outside, optical temperature sensors, organic solar cells (OSCs), organic field-effect transistors, organic diodes, organic photodiodes and down conversion systems.

## Revendications

1. Composition comprenant

   - une molécule organique pour l'émission de lumière, qui présente une valeur $\Delta E(S_1\text{-}T_1)$ entre l'état singulet excité le plus bas ($S_1$) et l'état triplet sous-jacent ($T_1$) inférieure à 3 000 $cm^{-1}$ et
   - un atome ou une molécule pour la réduction de la constante temporelle de conversion inter-système de la molécule organique à moins de 300 ms,

   la molécule organique présentant une structure de formule V

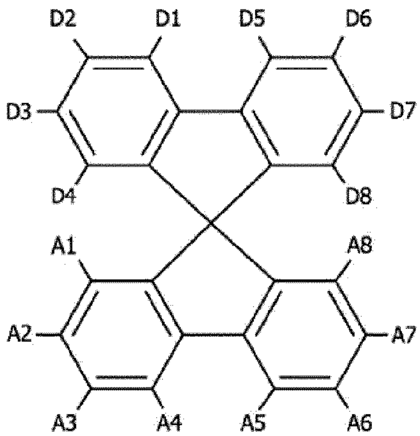

Formule V

   dans laquelle

   D1 à D8 représentent indépendamment les uns des autres H, Br, I et/ou des groupes qui représentent un donneur D ;
   les groupes donneurs peuvent être identiques ou différents ;
   au moins un groupe donneur est présent ;
   des groupes voisins Di, Dj et Dk de D1 à D8 étant des cycles aromatiques ou hétéroaromatiques en option conjugués, ces derniers présentant en option une fonction de donneur ;
   A1 à A8 représentent indépendamment les uns des autres H, Br, I et/ou des groupes qui représentent un accepteur A ;
   des groupes voisins Ai, Aj et Ak de A1 à A8 étant des cycles aromatiques ou hétéroaromatiques en option conjugués, ces derniers présentant en option une fonction d'accepteur ;
   les groupes accepteurs étant identiques ou différents ; et
   au moins un groupe accepteur étant présent ; et
   Br et/ou I étant en option lié(s) par l'intermédiaire d'un groupe alkyle (où C = 1 ou 2) à la molécule organique de formule V ;
   l'atome ou la molécule supplémentaire utilisé(e) pour la réduction de la constante temporelle de conversion inter-système de la molécule émettrice étant choisi(e) dans le groupe constitué par : les gaz rares krypton et xénon, des substances comportant du brome, des substances comportant de l'iode, des atomes métalliques, des nanoparticules métalliques, des ions métalliques, des complexes de Gd et des complexes de plomb.

2. Composition selon la revendication 1, dans laquelle l'atome ou la molécule supplémentaire ou des parties de molécule utilisé(e)(s) pour la réduction de la constante temporelle de conversion inter-système de la molécule émettrice présente(nt) une constante de couplage spin-orbite supérieure à 1 000 $cm^{-1}$, de préférence supérieure à 3 000 $cm^{-1}$, de façon particulièrement préférée supérieure à 4 000 $cm^{-1}$.

3. Composition selon la revendication 1 ou 2, dans laquelle la molécule organique dans la composition présente une valeur $\Delta E(S_1\text{-}T_1)$ entre l'état singulet excité le plus bas et l'état triplet sous-jacent inférieure à 2 500 $cm^{-1}$, de préférence inférieure à 1 500 $cm^{-1}$, de façon particulièrement préférée inférieure à 1 000 $cm^{-1}$.

4. Composition selon l'une quelconque des revendications 1 à 3, dans laquelle l'atome ou la molécule supplémentaire utilisé(e) pour la réduction de la constante temporelle de conversion inter-système de la molécule émettrice ne présente pas d'absorptions ou d'émissions dans la plage d'émission et/ou dans la plage HOMO/LUMO de la molécule organique.

5. Composition selon l'une quelconque des revendications 1 à 4, dans laquelle le rapport numérique entre l'atome ou la molécule utilisé(e) pour la réduction de la constante temporelle de conversion inter-système de la molécule émettrice et les molécules émettrices vaut de 1 : 0,1 à 1 : 50.

6. Composition selon l'une quelconque des revendications 1 à 5, dans laquelle l'atome ou la molécule destiné(e) à la réduction de la constante temporelle de conversion inter-système de la molécule organique est lié(e) par covalence à la molécule organique, et l'atome ou la molécule destiné(e) à la réduction de la constante temporelle de conversion inter-système, lié(e) par covalence à la molécule organique, provoque une augmentation du couplage spin-orbite.

7. Composition selon la revendication 6, dans laquelle l'atome ou la molécule destiné(e) à la réduction de la constante temporelle de conversion inter-système de la molécule organique, lié(e) par covalence à la molécule organique, est un atome d'iode et/ou un atome de brome.

8. Composition selon l'une quelconque des revendications 1 à 7, dans laquelle la molécule organique comporte au moins un atome de deutérium.

9. Composition selon l'une quelconque des revendications 1 à 7, dans laquelle la molécule organique est incorporée dans une matrice ou réticulée avec elle, la matrice comportant en option au moins un atome de deutérium.

10. Composition selon la revendication 9, dans laquelle la matrice est une matrice polymère ou une matrice réticulée en polymère, la matrice étant attachée à la molécule organique par liaison covalente.

11. Composition selon la revendication 9 ou 10, dans laquelle la matrice comporte au moins un additif lié par covalence, augmentant le couplage spin-orbite et abaissant ainsi la constante temporelle de conversion inter-système, en particulier un atome de brome ou un atome d'iode.

12. Composition comportant

- une matrice, comportant du ou consistant en glucose et/ou tréhalose et
- une composition selon l'une quelconque des revendications 1 à 8,

en particulier pour l'utilisation dans un dispositif optoélectronique.

13. Utilisation d'une composition selon l'une quelconque des revendications 1 à 9 dans une couche émettrice dans un dispositif optoélectronique.

14. Procédé pour la fabrication d'un dispositif optoélectronique, dans lequel on utilise une composition selon l'une quelconque des revendications 1 à 12.

15. Dispositif optoélectronique, comportant une composition selon les revendications 1 à 12.

16. Dispositif optoélectronique selon la revendication 15,
dans lequel la proportion de la composition dans une couche émettrice vaut de 0,5 % à 100 % en poids, de préférence de 6 à 30 % en poids, par rapport au poids total de la couche émettrice.

17. Dispositif optoélectronique organique selon la revendication 15 ou 16 sous forme d'une diode électroluminescente organique (OLED), **caractérisé par** une couche émettrice présentant la composition selon l'une quelconque des revendications 1 à 12, la proportion de la composition dans la couche émettrice étant comprise entre 0,5 % et 100 % en poids, de préférence entre 6 et 30 % en poids, par rapport au poids total de la couche émettrice.

18. Utilisation selon la revendication 13, dans laquelle le dispositif optoélectronique est choisi dans le groupe constitué par les diodes électroluminescentes organiques (OLED), les cellules électrochimiques photoémettrices (LEEC ou LEC), les capteurs OLED, en particulier les capteurs de gaz et de vapeur non isolés hermétiquement de l'extérieur,

les capteurs de température optiques, les cellules solaires organiques (CSO), les transistors à effet de champ organiques, les diodes organiques, les photodiodes organiques et les systèmes convertisseurs-abaisseurs.

**19.** Procédé selon la revendication 14, dans lequel le dispositif optoélectronique est choisi dans le groupe constitué par les diodes électroluminescentes organiques (OLED), les cellules électrochimiques photoémettrices (LEEC ou LEC), les capteurs OLED, en particulier les capteurs de gaz et de vapeur non isolés hermétiquement de l'extérieur, les capteurs de température optiques, les cellules solaires organiques (CSO), les transistors à effet de champ organiques, les diodes organiques, les photodiodes organiques et les systèmes convertisseurs-abaisseurs.

**20.** Dispositif optoélectronique selon l'une quelconque des revendications 15 à 17, où le dispositif optoélectronique est choisi dans le groupe constitué par les diodes électroluminescentes organiques (OLED), les cellules électrochimiques photoémettrices (LEEC ou LEC), les capteurs OLED, en particulier les capteurs de gaz et de vapeur non isolés hermétiquement de l'extérieur, les capteurs de température optiques, les cellules solaires organiques (CSO), les transistors à effet de champ organiques, les diodes organiques, les photodiodes organiques et les systèmes convertisseurs-abaisseurs.

**Figur 1**

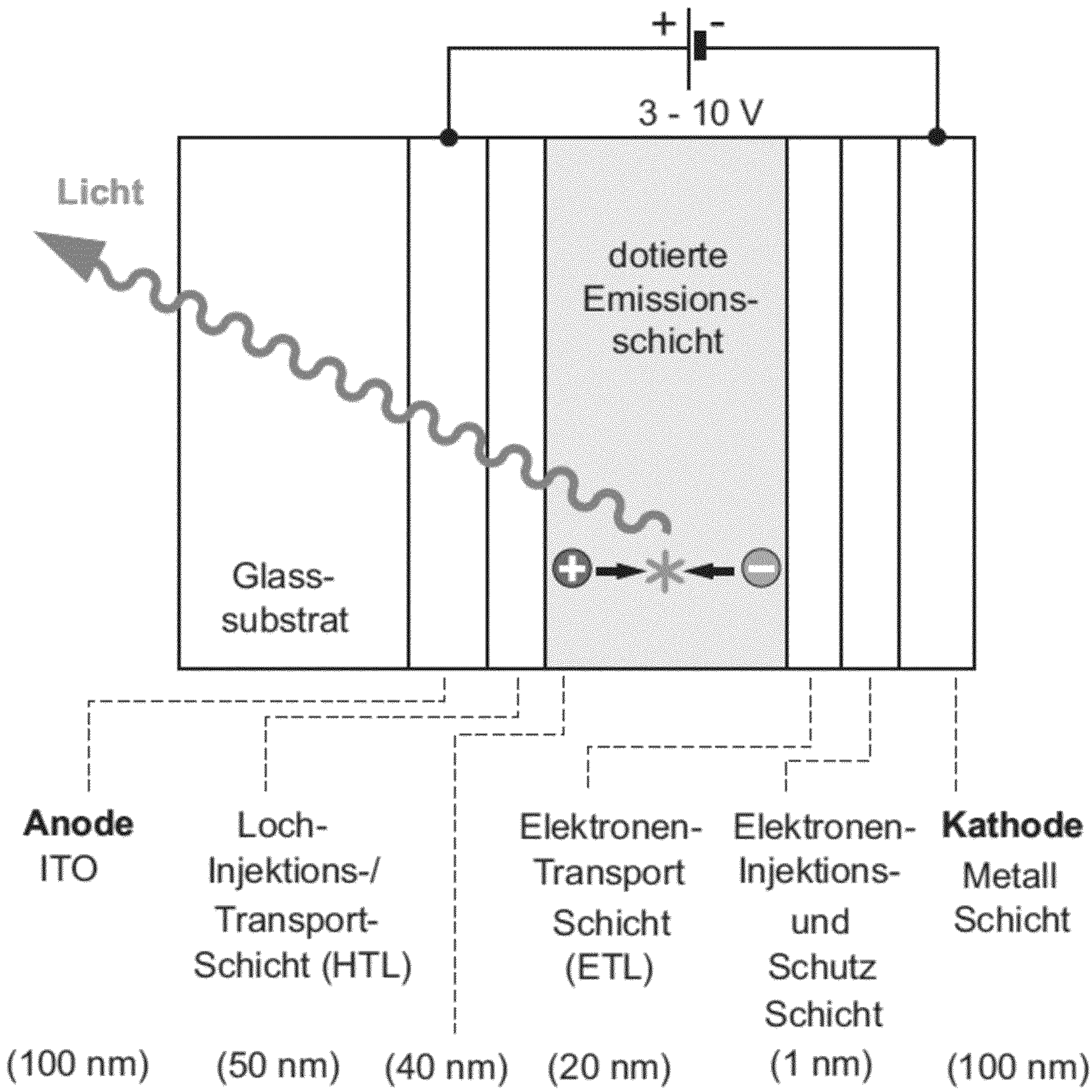
+
-
3 - 10 V
Licht
dotierte Emissions-schicht
Glass-substrat
Anode ITO
Loch-Injektions-/Transport-Schicht (HTL)
Elektronen-Transport Schicht (ETL)
Elektronen-Injektions- und Schutz Schicht
Kathode Metall Schicht
(100 nm)
(50 nm)
(40 nm)
(20 nm)
(1 nm)
(100 nm)

**Figur 2**

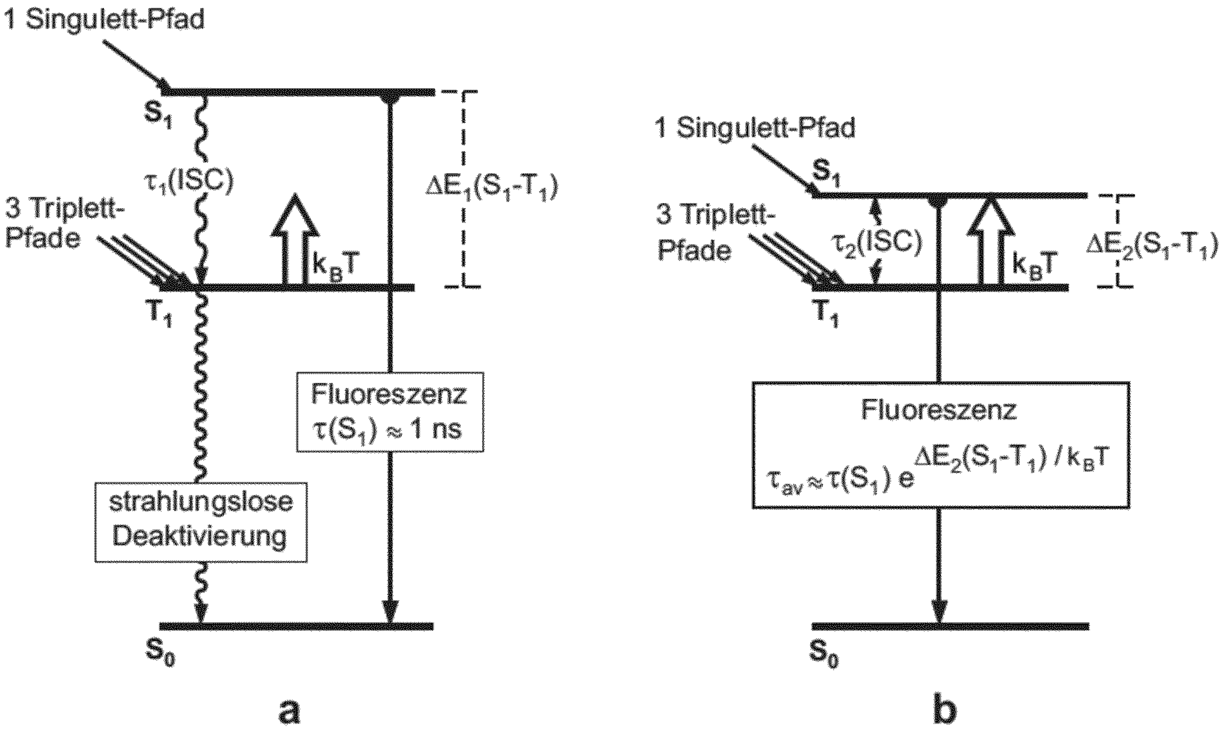


**Figur 3**

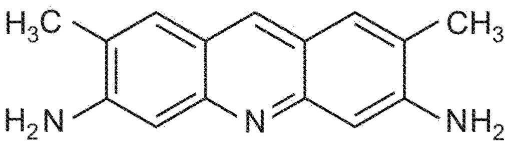

Acridin Gelb

**Figur 4**

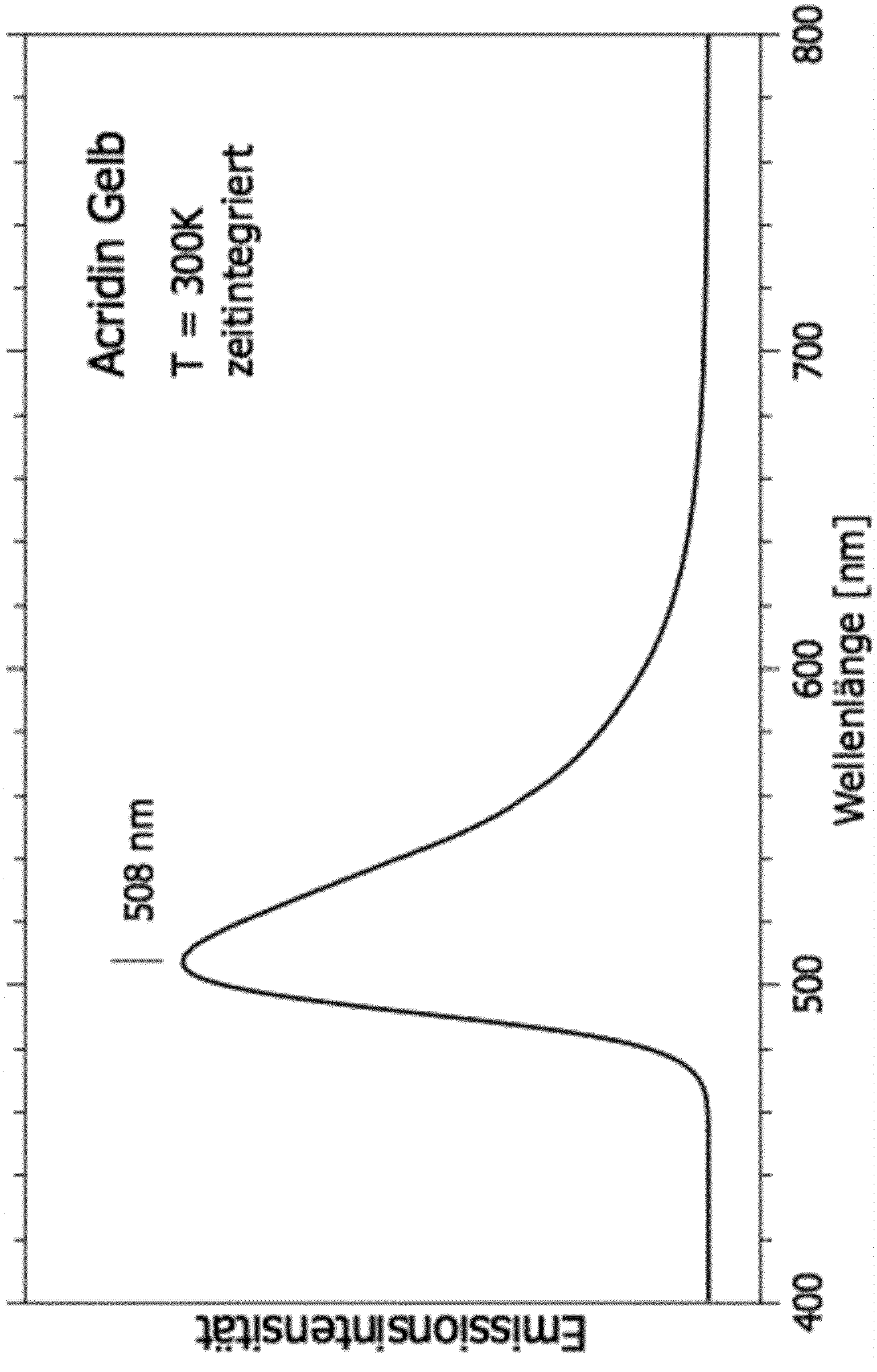
Acridin Gelb
T = 300K
zeitintegriert
508 nm
400
500
600
700
800
Wellenlänge [nm]
Emissionsintensität

Figur 5

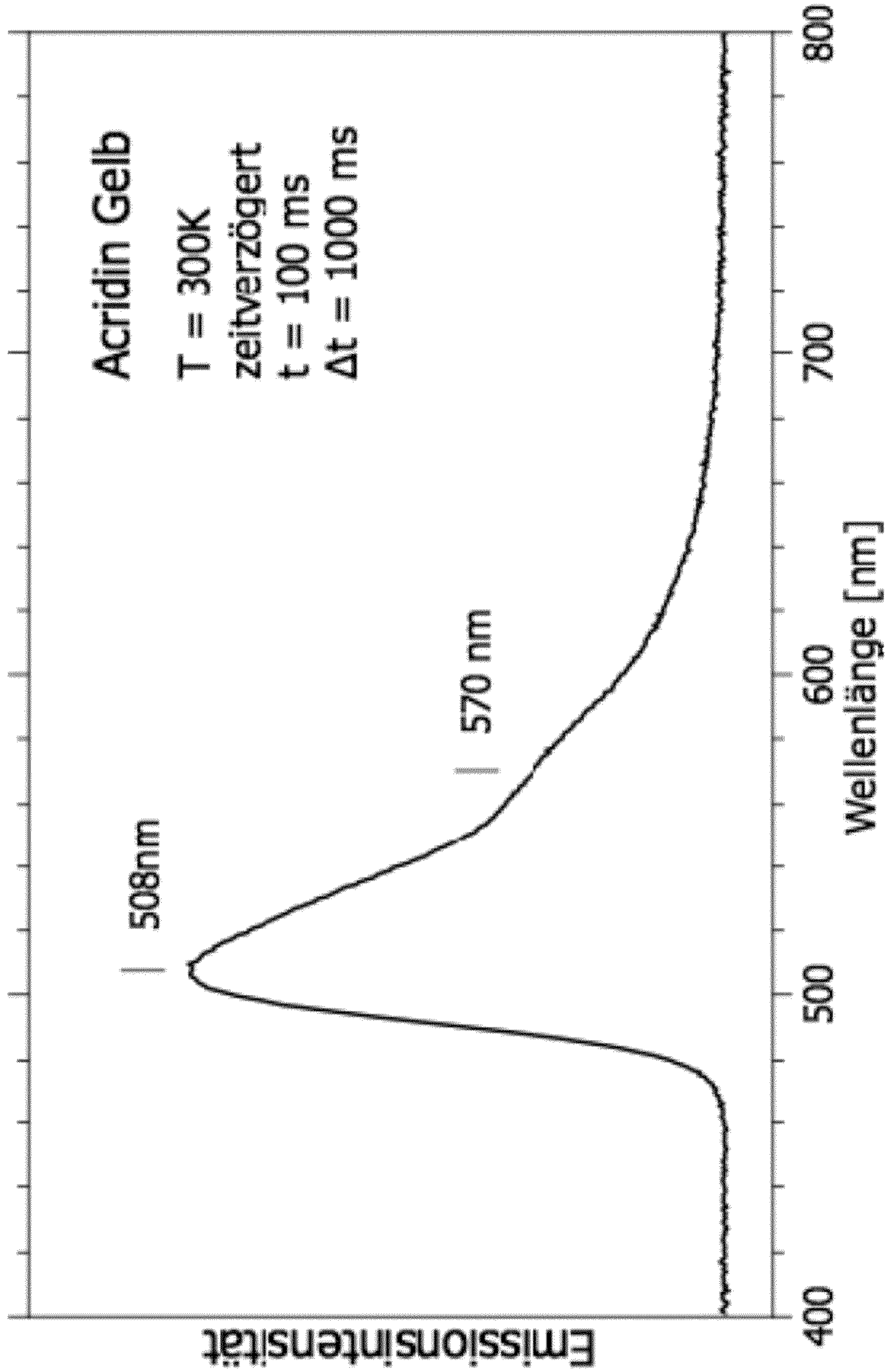
Acridin Gelb
T = 300K
zeitverzögert
t = 100 ms
Δt = 1000 ms
508nm
570 nm
Emissionsintensität
Wellenlänge [nm]
400
500
600
700
800

Figur 6

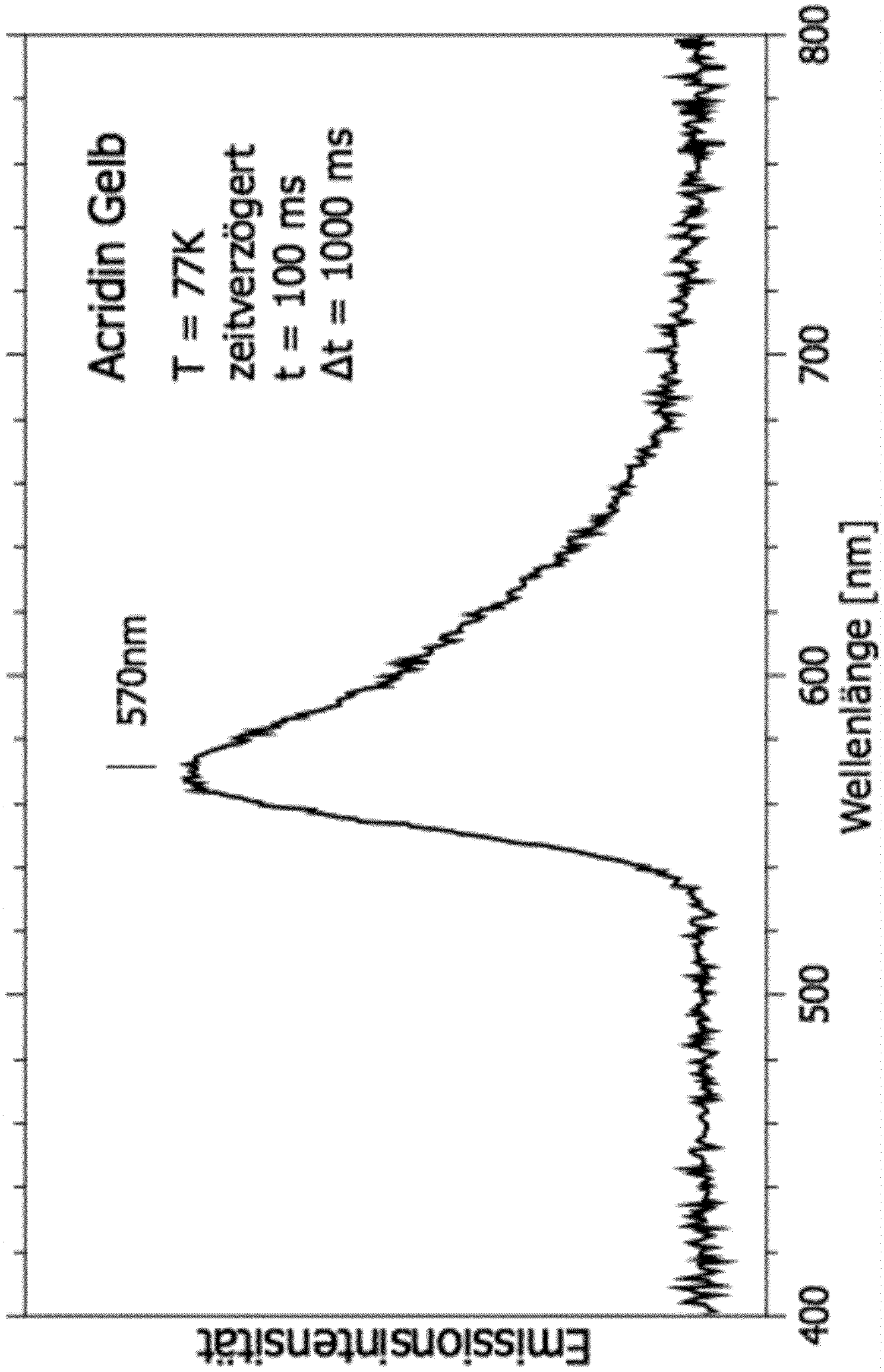
Acridin Gelb
T = 77K
zeitverzögert
t = 100 ms
Δt = 1000 ms
570nm
Emissionsintensität
Wellenlänge [nm]
400
500
600
700
800

**Figur 7**

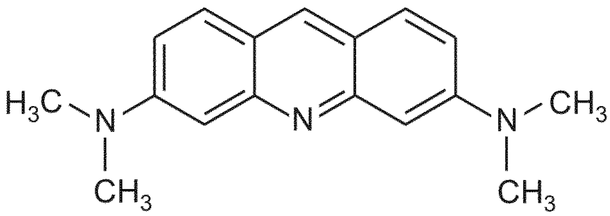

Acridin Orange

Figur 8

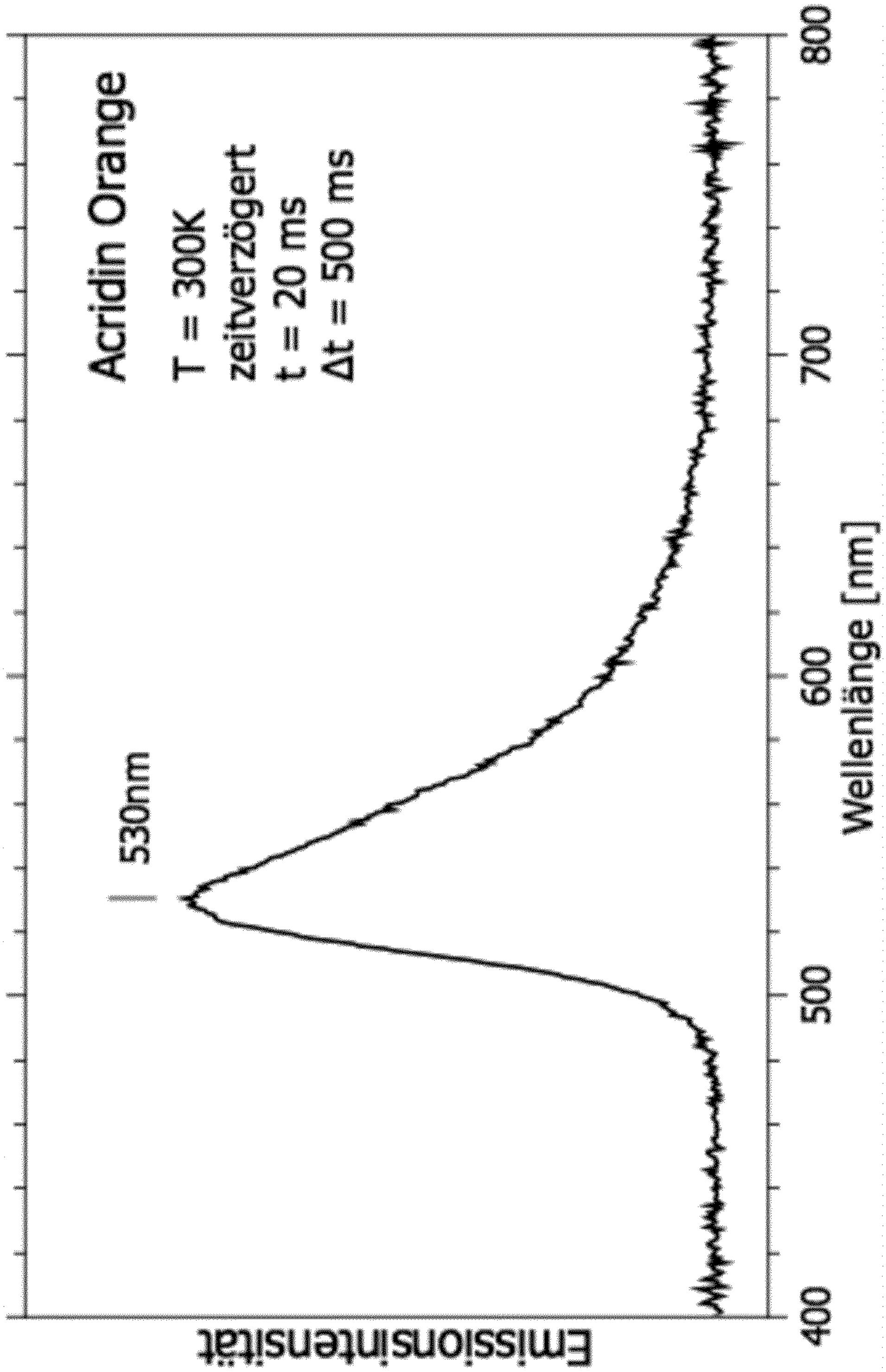
Acridin Orange
T = 300K
zeitverzögert
t = 20 ms
Δt = 500 ms
530nm
400
500
600
700
800
Wellenlänge [nm]
Emissionsintensität

Figur 9

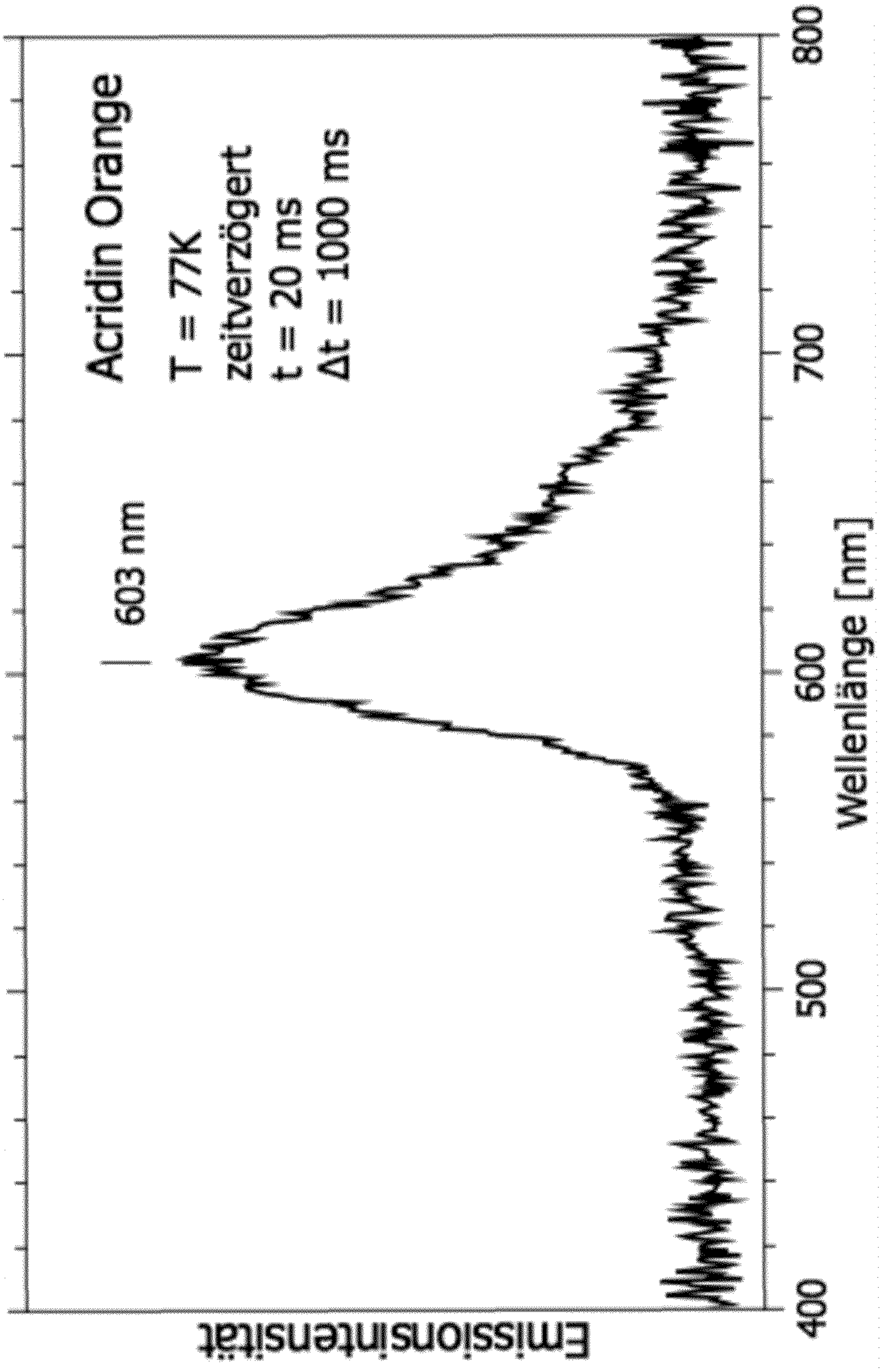
Acridin Orange
T = 77K
zeitverzögert
t = 20 ms
Δt = 1000 ms
603 nm
Emissionsintensität
Wellenlänge [nm]
400
500
600
700
800

**Figur 10**

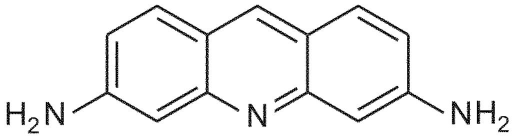

Proflavin

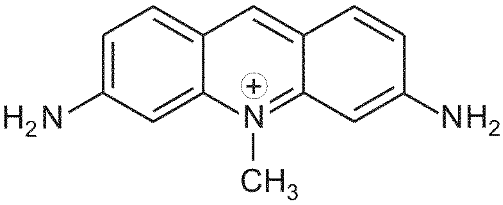

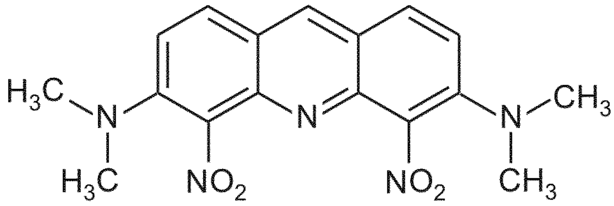

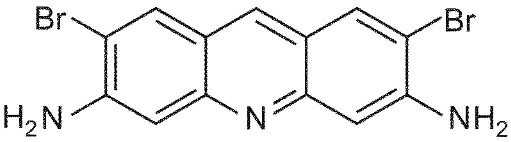

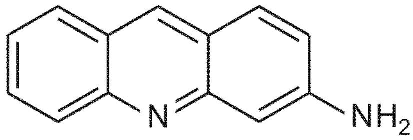

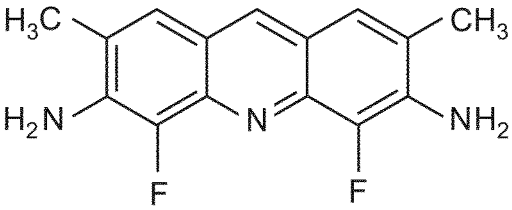

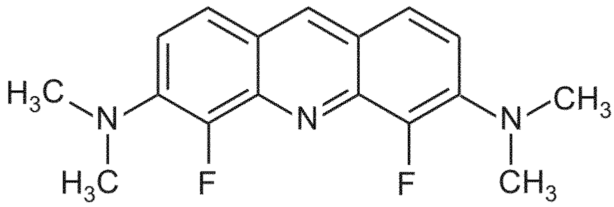

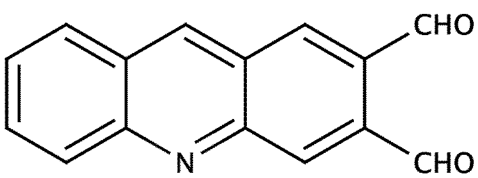

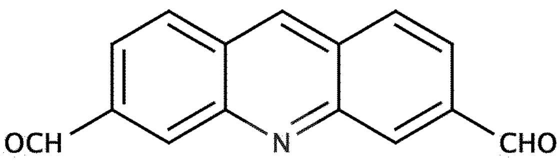

**Figur 11**

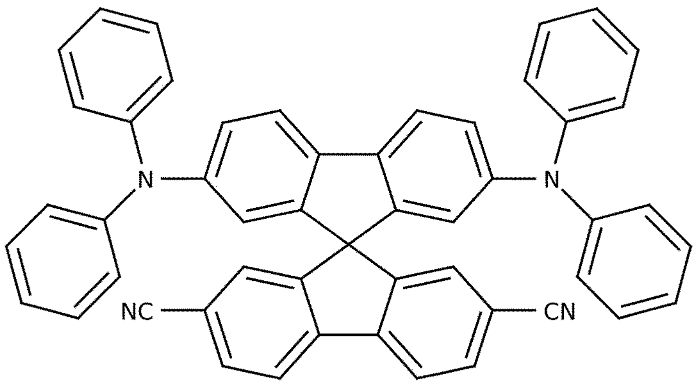

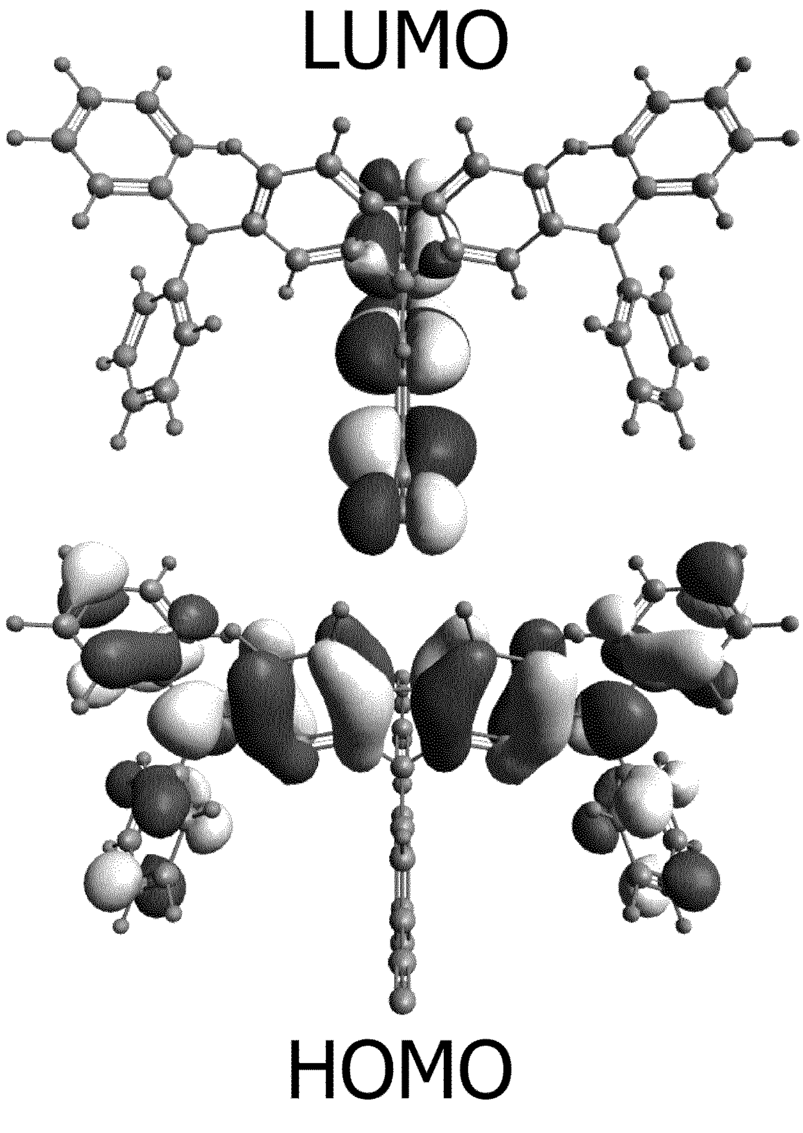

Figur 12

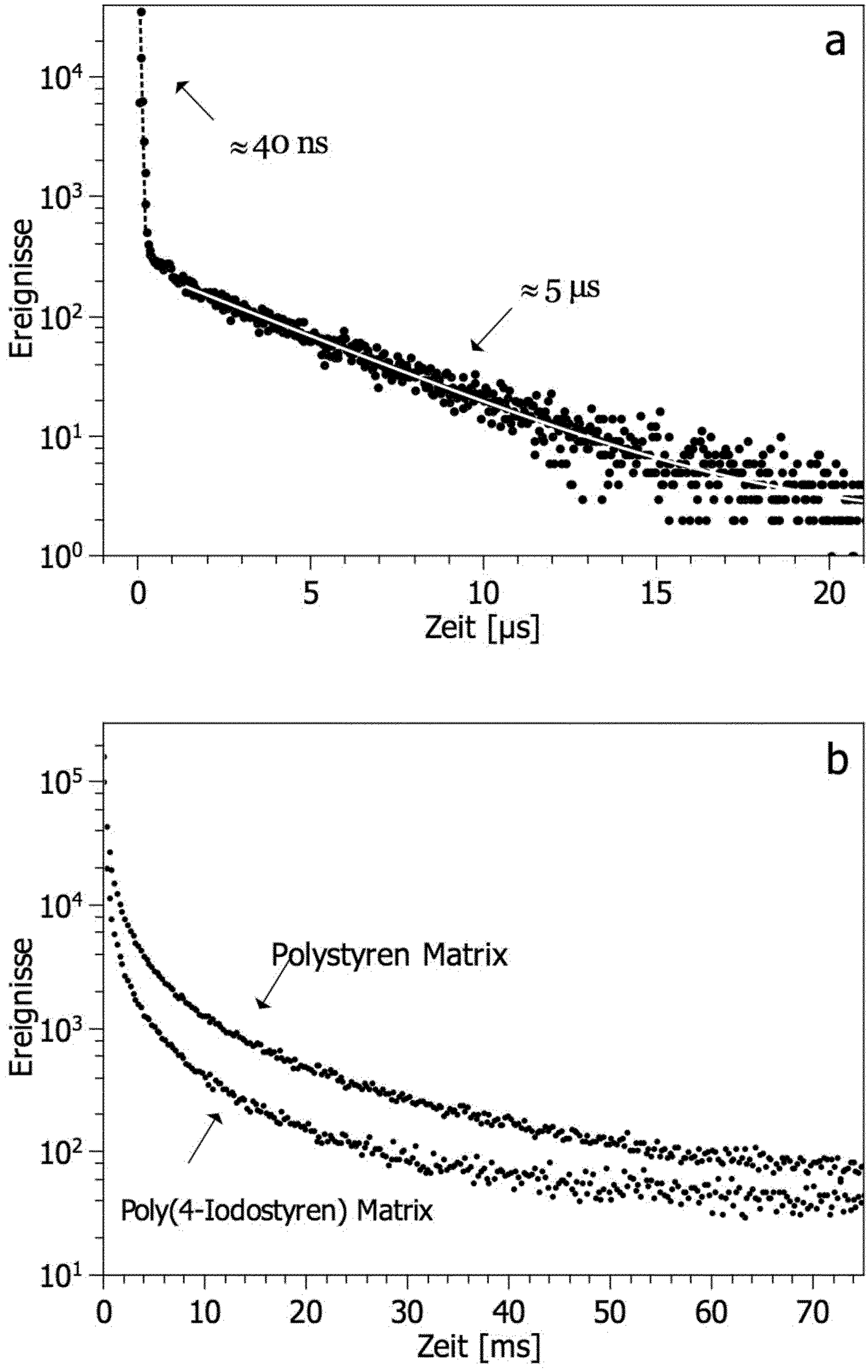
a
≈40 ns
≈5 µs
Ereignisse
$10^0$
$10^1$
$10^2$
$10^3$
$10^4$
0
5
10
15
20
Zeit [µs]
b
Polystyren Matrix
Poly(4-Iodostyren) Matrix
Ereignisse
$10^1$
$10^2$
$10^3$
$10^4$
$10^5$
0
10
20
30
40
50
60
70
Zeit [ms]

**Figur 13**

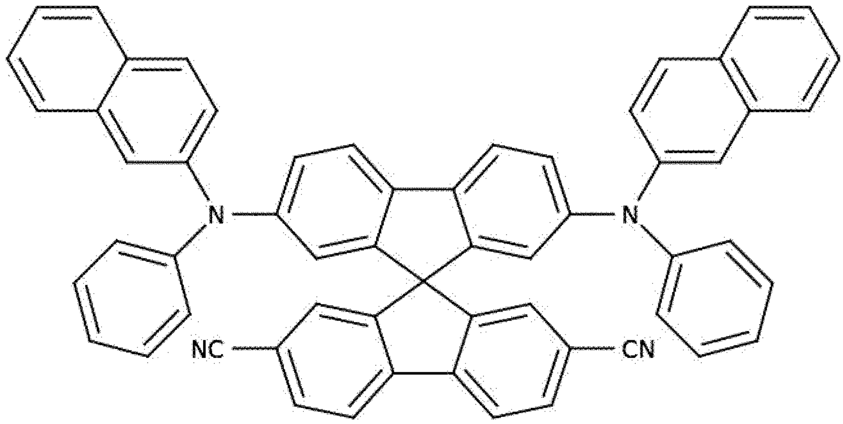

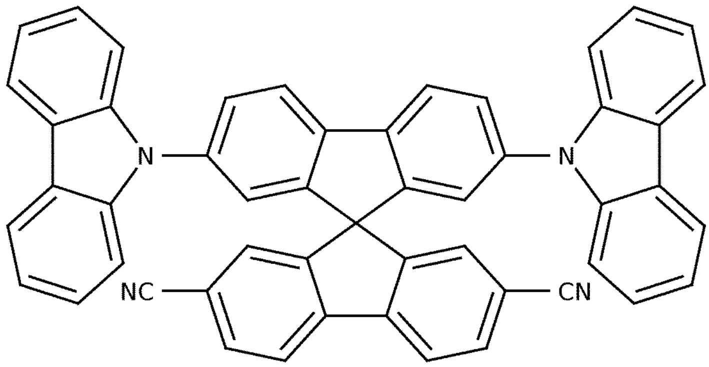

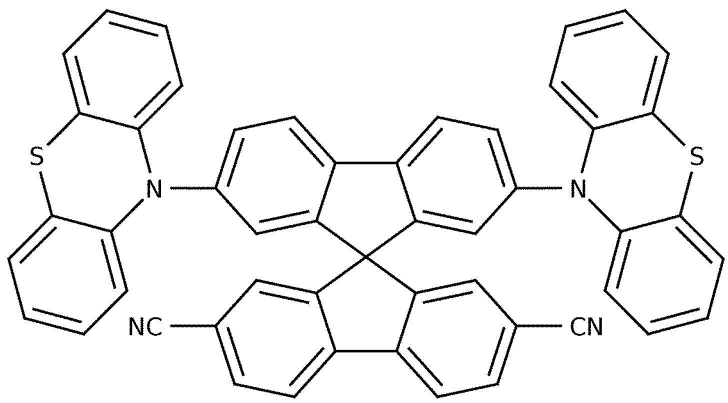

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **H. YERSIN.** *Top. Curr. Chem.,* 2004, vol. 241, 1
- **H. YERSIN.** Highly Efficient OLEDs with Phosphorescent Materials. Wiley-VCH, 2008
- **TANG et al.** *Appl. Phys. Lett.,* 1987, vol. 51, 913
- **M. A. BALDO ; D. F. O'BRIEN ; M. E. THOMPSON ; S. R. FORREST.** *Phys. Rev. B,* 1999, vol. 60, 14422
- **T. SAJOTO ; P. I. DJUROVICH ; A. B. TAMAYO ; J. OXGAARD ; W. A. GODDARD III ; M. E. THOMPSON.** *J. Am. Chem. Soc.,* 2009, vol. 131, 9813
- **WEN-YI HUNG et al.** *Physical Chemistry Chemical Physics,* 01. Januar 2008, vol. 10 (38), 5822-5825
- **X. XING et al.** *Organic Electronics,* 2012, vol. 13, 195-198
- **J. B. BIRKS.** Photophysics of Aromatic Molecules. Wiley-Interscience, 1970
- **E. LIPPERT.** Z Naturforsch. 1955, vol. 10a, 541
- **C. A. ZUNIGA ; S. BARLOW ; S. R. MARDER.** *Chem. Materials,* 2011, vol. 23, 658-681